# EUROPEAN PATENT APPLICATION

(11) **EP 4 282 867 A1**
(43) Date of publication of application: **29.11.2023**
(21) Application number: 22794789.2
(22) Date of filing: 24.04.2022
(51) Int. Cl.: C07D 471/10, A61K 31/438, A61P 3/00

(54) **INDOLINE-CONTAINING SPIRO DERIVATIVE, PREPARATION METHOD THEREFOR AND APPLICATION THEREOF IN MEDICINE**

(30) Priority: 25.04.2021 CN 202110450480
(71) Applicant: Changchun Genescience Pharmaceutical Co., Ltd., Changchun, Jilin 130012 (CN)
(72) Inventor: SONG, Yunlong, Changchun, Jilin 130012 (CN); SHEN, Guangyuan, Changchun, Jilin 130012 (CN); LIU, Peng, Changchun, Jilin 130012 (CN); JIN, Lei, Changchun, Jilin 130012 (CN); WANG, Guocheng, Changchun, Jilin 130012 (CN); LIN, Daizong, Changchun, Jilin 130012 (CN); TENG, Guosheng, Changchun, Jilin 130012 (CN); YAN, Hongbo, Changchun, Jilin 130012 (CN)
(74) Representative: Gille Hrabal Partnerschaftsgesellschaft mbB Patentanwälte
(86) International application number: PCT/CN2022/088656
(87) International publication number: WO 2022/228318

(57) **Abstract**

The present invention relates to a compound of Formula I, or a racemate, a racemate, a stereoisomer, a tautomer, an isotopically labeled compound, an N-oxide, a solvate, a polymorph, a metabolite, an ester, a prodrug or a pharmaceutically acceptable salt thereof, and a pharmaceutical composition comprising the same, a preparation method therefor, and use thereof as a GHSR agonist in the preparation of drugs for diagnosing, preventing and/or treating growth hormone dependent diseases. The structure of Formula I is shown below:

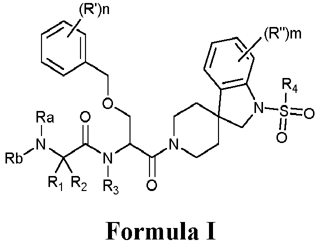

## Description

### CROSS-REFERENCE TO RELATED APPLICATION

The present application claims priority of preceding Patent Application No. 202110450480.1 filed with State Intellectual Property Office of the PRC on April 25, 2021, and entitled "Spiroindoline derivative, and preparation method and medical use thereof, which is incorporated herein by reference in its entirety.

### TECHNICAL FIELD

The present invention relates to the technical field of drug compounds, and particularly to a spiroindoline derivative, and a preparation method and medical use thereof.

### BACKGROUND

Ghrelin is an endogenous growth hormone releasing peptide with 28 amino acids, and an endogenous ligand for growth hormone secretagogue receptor 1a (GHSR 1a). Both in-vivo and in-vitro tests have confirmed that Ghrelin can significantly promote the secretion of growth hormone (GH). Clinical studies have also found that intravenous injection of Ghrelin can potently stimulate the release of growth hormone in a dose-dependent manner.

Human growth hormone is a peptide hormone consisting of 191 amino acids secreted by the anterior lobe of the pituitary gland. By inducing the synthesis of insulin-like growth factor-1 (IGF-1) or epidermal growth factor (EGF), it directly or indirectly acts on peripheral organs. Human growth hormone has main physiological functions including promoting the linear growth of the body, promoting the cell proliferation of muscles and skin, and playing an important role in tissue regeneration after trauma.

The release of GH is considered to be useful in the treatment of physiological or pathophysiological diseases characterized by growth hormone deficiency, and diseases able to be improved by the anabolic effect of growth hormone. Clinically, GH is expected to treat of muscle loss, fat accumulation, bone demineralization, decreased tissue regeneration ability after injury, and other disorders.

GH is synthesized and stored in pituitary gland, but the release of GH is controlled by hypothalamic hormones. At present, two hormones are known to participate in the release process of GH, including a growth hormone releasing hormone (GHRH) and a somatotropin release inhibiting hormone (SRIF). In most cases, GH deficiency involves GH release (hypothalamic dysfunction) rather than GH synthesis (pituitary dysfunction). Therefore, the use of a GHSR agonist to stimulate the GH release in pituitary gland may be a new therapy to replace recombinant human growth hormone.

GHSR has two subtypes, 1a and 1b. Subtype 1a is a functional receptor subtype, and the function of Subtype 1b needs further study. In the central nervous system, GHS-R1a is distributed in the hypothalamus and other areas, including the pituitary, and the arcuate nucleus and ventromedial nucleus of hypothalamus. In the periphery, GHSR is also lowly expressed in thyroid, pancreas and myocardium. Therefore, Ghrelin and its receptor GHSR1a may be involved in the regulation of various functions in the body.

Studies find that some peptides or peptide-like clinical compounds show GHSR agonist activity and induce GH release. At present, compounds entering clinical studies include examorelin, tabimorelin, pralmorelin, ibutamoren, tesamorelin, anamorelin, and macimorelin. Among them, the injections of polypeptide tesamorelin (used to reduce excess belly fat in patients infected with HIV) and small peptide-like macimorelin have been approved for marketing by FDA. Macimorelin is the only oral drug approved for the diagnosis of adult growth hormone deficiency, but it also has some defects such as poor oral bioavailability and potential risk of cardiotoxicity.

In related studies, it is also found that GHSR agonists not only mediate and induce the GH secretion through GHSR1a activation, but also mediate other physiological functions through various receptors in other GHS receptor families or different binding sites on GHSR (such as GHSR1b, motilin receptor type 1a, neurotensin receptor and TRH receptor). Therefore, the use of GHSR agonists in gastrointestinal indications has been newly developed. At present, there are no drugs for these indications available on the market. Ulimorelin and relamorelin have entered the phase III clinical trial.

Ghrelin has been confirmed to promote gastrointestinal peristalsis through vagus nerve and pelvic nerve. However, the short half-life of Ghrelin hinders its druggability. Therefore, there is a need to develop a GHSR agonist with enhanced pharmacokinetics to improve gastrointestinal dysfunction in animals and humans. Studies find that ibutamoren shows good safety in clinical trials, and has a GHSR agonist activity. However, it does not reach the clinical end point after long-term administration. Therefore, a GHSR agonist developed based on ibutamoren will have a good prospect in clinical application while the potential defects are avoided.

### SUMMARY

To solve the problems existing in the prior art, the present invention provides a compound of Formula I, and a racemate, a stereoisomer, a tautomer, an isotopically labeled compound, an N-oxide, a solvate, a polymorph, a metabolite, an ester, a prodrug or a pharmaceutically acceptable salt thereof: 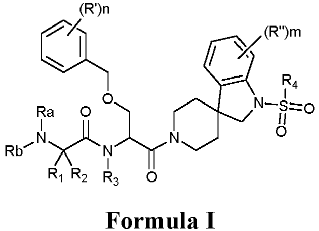 where
Ra is selected from H, -R, -C(=O)R or -C(=O)OR;
Rb is selected from H, -R, -C(=O)R or -C(=O)OR;
or Rb is attached to N in N-R₃ to form a ring structure 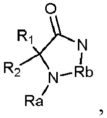 and in this case R₃ is absent, Rb is selected from 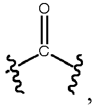 or a (C₁-C₁₂) aliphatic hydrocarbylene group optionally substituted with one, two or more Rc, and Ra is as defined above;
provided that Ra and Rb are not both H;
R is selected from a (C₁-C₂₀) aliphatic hydrocarbyl group, a (C₁-C₂₀) aliphatic hydrocarbyl group optionally containing one, two or more heteroatoms, a C₃-₁₂ cycloalkyl group, a 3-12 membered heterocyclyl group, a C₆₋₁₄ aryl group or a 5-14 membered heteroaryl group, optionally substituted with one, two or more Rc;
Rc is each independently selected from halo, CN, OH, SH, =O (oxo), NH₂, -OP(=O)(OH)₂, -OP(=O)(C₁-C₁₂ alkoxy)₂, COOH, or a (C₁-C₁₂) aliphatic hydrocarbyl group, a (C₁-C₁₂) aliphatic hydrocarbyl group optionally containing one, two or more heteroatoms, a C₃₋₁₂ cycloalkyl group, a 3-12 membered heterocyclyl group, a C₆₋₁₄ aryl group or a 5-14 membered heteroaryl group, optionally substituted with one, two or more Rd;
Rd is each independently selected from halo, CN, OH, SH, =O (oxo), NH₂, -OP(=O)(OH)₂, -OP(=O)(C₁-C₁₂ alkoxy)₂, COOH, a (C₁-C₁₂) aliphatic hydrocarbyl group, and a (C₁-C₁₂) aliphatic hydrocarbyl group optionally containing one, two or more heteroatoms;
R₁, R₂, R₃, and R₄ are the same or different, and each independently selected from H, and a (C₁-C₁₂) aliphatic hydrocarbyl group optionally substituted with one, two or more Rc;
R' and R" are each independently selected from H, halo, CN, OH, SH, NH₂,-OP(=O)(OH)₂, -OP(=O)(C₁-C₁₂ alkoxy)₂, COOH, or a (C₁-C₁₂) aliphatic hydrocarbyl group and a (C₁-C₁₂) aliphatic hydrocarbyl optionally containing one, two or more heteroatoms, optionally substituted with one, two or more Rd; and
n, and m are each independently selected from 0, 1, 2, 3, 4 or 5.

According to an embodiment of the present invention, in the "(C₁-C₂₀) aliphatic hydrocarbyl group optionally containing one, two or more heteroatoms", the heteroatom is selected from sulfur, nitrogen, oxygen, phosphorus and silicon, and optionally the heteroatom is interposed in an optional C-C bond and C-H bond in the aliphatic hydrocarbyl group.

The "(C₁-C₂₀) aliphatic hydrocarbyl group" can be selected from a (C₁-C₂₀) alkyl group, a (C₂-C₂₀) alkenyl group, and a (C₂-C₂₀) alkynyl group; the "(C₁-C₂₀) aliphatic hydrocarbyl group" can be selected from a (C₁-C₁₂) alkyl group, a (C₂-C₁₂) alkenyl group, and a (C₂-C₁₂) alkynyl group; and the "(C₁-C₁₂) aliphatic hydrocarbyl group" can be selected from a (C₁-C₆) alkyl group, a (C₂-C₆) alkenyl group, and a (C₂-C₆)alkynyl group.

The "halo" is selected from F, Cl, Br, and I.

According to an embodiment of the present invention,
Ra is selected from H;
Rb is selected from -R, -C(=O)R or -C(=O)OR;
or Rb is attached to N in N-R₃ to form a ring structure 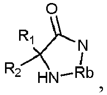 and in this case, R₃ is absent, Rb is selected from 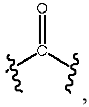 or a (C₁-C₁₂) alkylene group and a (C₁-C₁₂) alkenylene group optionally substituted with one, two or more Rc;
R is selected from a (C₁-C₂₀) alkyl group, a (C₁-C₂₀) alkenyl group, a (C₁-C₂₀) alkyl group or a (C₁-C₂₀) alkenyl group optionally containing one, two or more heteroatoms, a C₃₋₁₂ cycloalkyl group, a 3-12 membered heterocyclyl group, a C₆₋₁₄ aryl group or a 5-14 membered heteroaryl group, optionally substituted with one, two or more Rc;
Rc is each independently selected from halo, CN, OH, SH, =O(oxo), NH₂, -OP(=O)(OH)₂, -OP(=O)(C₁-C₁₂ alkoxy)₂, COOH, or a (C₁-C₁₂) alkyl group, a (C₁-C₁₂) alkenyl group, a (C₁-C₁₂) alkyl group or (C₁-C₁₂) alkenyl group optionally containing one, two or more heteroatoms, a C₃₋₁₂ cycloalkyl group, a 3-12 membered heterocyclyl group, a C₆₋₁₄ group or a 5-14 membered heteroaryl group, optionally substituted with one, two or more Rd;
Rd is each independently selected from halo, CN, OH, SH, =O (oxo), NH₂, -OP(=O)(OH)₂, -OP(=O)(C₁-C₁₂ alkoxy)₂, COOH, a (C₁-C₁₂) alkyl group, a (C₁-C₁₂) alkenyl group, and a (C₁-C₁₂) alkyl group or a (C₁-C₁₂) alkenyl group optionally containing one, two or more heteroatoms;
R₁, R₂, R₃, and R₄ are the same or different, and each independently selected from H, and a (C₁-C₁₂) alkyl group or (C₁-C₁₂) alkenyl group optionally substituted with one, two or more Rc;
R' and R" are each independently selected from H, halo, CN, OH, SH, NH₂,-OP(=O)(OH)₂, -OP(=O)(C₁-C₁₂ alkoxy)₂, COOH, or a (C₁-C₁₂) alkyl group, a (C₁-C₁₂) alkenyl group, and a (C₁-C₁₂) alkyl group or a (C₁-C₁₂) alkenyl group optionally containing one, two or more heteroatoms, optionally substituted with one, two or more Rd; and
n, and m are each independently selected from 0, 1, 2, 3, 4 or 5.
According to an embodiment of the present invention,
Ra is selected from H;
Rb is selected from -R, -C(=O)R or -C(=O)OR;
or Rb is attached to N in N-R₃ to form a ring structure 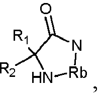 and in this case R₃ is absent, Rb is selected from 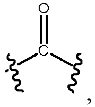 or a (C₁-C₆) alkylene group and a (C₁-C₆) alkenylene group optionally substituted with one, two or more Rc;
R is selected from a (C₁-C₂₀) alkyl group, a (C₁-C₂₀) alkenyl group, a (C₁-C₁₂) alkyl group or a (C₁-C₂₀) alkenyl group optionally containing one, two or more oxygen atoms, a C₃-₈ cycloalkyl group, a 3-8 membered heterocyclyl group, and a C₆₋₁₀ aryl group or a 5-9 membered heteroaryl group, optionally substituted with one, two or more Rc;
Rc is each independently selected from halo, CN, OH, SH, =O(oxo), NH₂, -OP(=O)(OH)₂, -OP(=O)(C₁-C₆ alkoxy)₂, COOH, or a (C₁-C₆) alkyl group, a (C₁-C₆) alkenyl group, a (C₁-C₆) alkyl group or a (C₁-C₆) alkenyl group optionally containing one, two or more oxygen atoms, a C₃₋₈ cycloalkyl group, a 3-8 membered heterocyclyl group, and a C₆₋₁₀ aryl group or a 5-9 membered heteroaryl group, optionally substituted with one, two or more Rd;
Rd is each independently selected from halo, CN, OH, SH, =O(oxo), NH₂, -OP(=O)(OH)₂,
-OP(=O)(C₁-C₁₂ alkoxy)₂, COOH, a (C₁-C₆) alkyl group or (C₁-C₆) alkenyl group, and a (C₁-C₆) alkyl group or a (C₁-C₆) alkenyl group optionally containing one, two or more oxygen atoms;
R₁, R₂, R₃, and R₄ are the same or different, and each independently selected from H, and a (C₁-C₆) alkyl group or a (C₁-C₆) alkenyl group optionally substituted with one, two or more Rc;
R' and R" are each independently selected from H, halo, CN, OH, SH, NH₂,-OP(=O)(OH)₂, -OP(=O)(C₁-C₁₂ alkoxy)₂, COOH, or a (C₁-C₆) alkyl group, a (C₁-C₆) alkenyl group, and a (C₁-C₆) alkyl group or a (C₁-C₆) alkenyl group optionally containing one, two or more heteroatoms , optionally substituted with one, two or more Rd; and
n, and m are each independently selected from 0, 1, 2, 3, 4 or 5.

According to an embodiment of the present invention,
Ra is selected from H;
Rb is selected from -R, -C(=O)R or -C(=O)OR; or Rb is attached to N in N-R₃ to form a ring structure 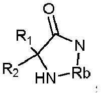 and in this case, R₃ is absent, Rb is selected from 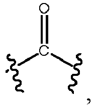 or a (C₁-C₁₂) alkylene group and a (C₁-C₁₂) alkenylene group optionally substituted with one, two or more Rc;
R is selected from a (C₁-C₂₀) alkyl group, a (C₁-C₂₀) alkenyl group, a (C₁-C₆) alkylOCH(C₁-C₆ alkyl)- group, a (C₁-C₁₂) alkylOCH₂- group, a C₃-₁₂ cycloalkyl group, a 3-12 membered heterocyclyl group, a C₆₋₁₄ aryl group or a 5-14 membered heteroaryl group, optionally substituted with one, two or more Rc; Rc is each independently selected from halo, CN, OH, SH, =O (oxo), NH₂, -OP(=O)(OH)₂,
-OP(=O)(C₁-C₁₂ alkoxy)₂, COOH, or a (C₁-C₁₂) alkyl group, a (C₁-C₁₂) alkenyl group, a (C₁-C₆) alkylOCH(C₁-C₆ alkyl)- group, a (C₁-C₁₂) alkylOCH₂- group, a C₃₋₁₂ cycloalkyl group, a 3-12 membered heterocyclyl group, a C₆₋₁₄ aryl group or a 5-14 membered heteroaryl group, optionally substituted with one, two or more Rd;
Rd is each independently selected from halo, CN, OH, SH, =O (oxo), NH₂, -OP(=O)(OH)₂, -OP(=O)(C₁-C₁₂ alkoxy)₂, COOH, a (C₁-C₁₂) alkyl group, a (C₁-C₁₂) alkenyl group, a (C₁-C₆) alkylOCH(C₁-C₆ alkyl)- group, and a (C₁-C₁₂) alkylOCH₂- group;
R₁, R₂, R₃, and R₄ are the same or different, and each independently selected from H, and a (C₁-C₁₂) alkyl group or a (C₁-C₁₂) alkenyl group optionally substituted with one, two or more Rc;
R' and R" are each independently selected from H, halo, CN, OH, SH, NH₂,-OP(=O)(OH)₂, -OP(=O)(C₁-C₁₂ alkoxy)₂, COOH, or a (C₁-C₁₂) alkyl group, a (C₁-C₁₂) alkenyl group, a (C₁-C₆) alkylOCH(C₁-C₆ alkyl)- group, and a (C₁-C₁₂) alkylOCH₂- group, optionally substituted with one, two or more Rd; and
n, and m are each independently selected from 0, 1, 2, 3, 4 or 5.
According to an embodiment of the present invention,
Ra is selected from H;
Rb is selected from -R, -C(=O)R or -C(=O)OR; or Rb is attached to N in N-R₃ (in this case, R₃ is absent) to form a ring structure 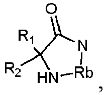 and in this case, Rb is selected from 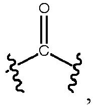 and a (C₁-C₆) alkylene group or a (C₁-C₆) alkenylene group optionally substituted with one, two or more Rc;
R is selected from a (C₁-C₂₀) alkyl group, a (C₁-C₂₀) alkenyl group, a (C₁-C₆) alkylOCH(C₁-C₆ alkyl)- group, a (C₁-C₁₂) alkylOCH₂-, C₃-₈ cycloalkyl group, a 3-8 membered heterocyclyl group, a C₆₋₁₀ aryl group or a 5-9 membered heteroaryl group, optionally substituted with one, two or more Rc;
Rc is each independently selected from halo, CN, OH, SH, =O (oxo), NH₂, -OP(=O)(OH)₂, -OP(=O)(C₁-C₆ alkoxy)₂, COOH, or a (C₁-C₆) alkyl group, a (C₁-C₆) alkenyl group, a (C₁-C₃) alkylOCH(C₁-C₃ alkyl)- group, a (C₁-C₃) alkylOCH₂- group, a C₃₋₈ cycloalkyl group, a 3-8 membered heterocyclyl group, a C₆₋₁₀ aryl group, or a 5-9 membered heteroaryl group, optionally substituted with one, two or more Rd;
Rd is each independently selected from halo, CN, OH, SH, =O (oxo), NH₂, -OP(=O)(OH)₂, -OP(=O)(C₁-C₆ alkoxy)₂, COOH, a (C₁-C₆) alkyl group or a (C₁-C₆) alkenyl group, a (C₁-C₃) alkylOCH(C₁-C₃ alkyl)- group, and a (C₁-C₃) alkylOCH₂- group;
R₁, R₂, R₃, and R₄ are the same or different, and each independently selected from H, and a (C₁-C₆) alkyl group or (C₁-C₆) alkenyl group optionally substituted with one, two or more Rc;
R' and R" are each independently selected from H, halo, CN, OH, SH, NH₂, - OP(=O)(OH)₂, -OP(=O)(C₁-C₆ alkoxy)₂, COOH, or a (C₁-C₆) alkyl group, a (C₁-C₆) alkenyl group, a (C₁-C₆) alkylOCH(C₁-C₆ alkyl)- group, and a (C₁-C₆) alkylOCH₂- group, optionally substituted with one, two or more Rd; and
n, and m are each independently selected from 0, 1, 2, 3, 4 or 5.

According to the present invention, in some embodiments, Ra is H;
Rb is selected from -R, -C(=O)R or -C(=O)OR;
R is selected from a (C₁-C₆) alkylC(=O)(C₁-C₆) alkyl- group, a HC(=O)(C₁-C₁₂) alkyl-group, a (C₁-C₁₂) alkylC(=O)OCH(C₁-C₁₂ alkyl)- group, a (C₁-C₁₂) alkyl- group, and a 5-14 membered heteroaryl-(C₁-C₁₂) alkyl- group, optionally substituted with one, two, or more NH₂;
or R is selected from a (C₁-C₁₂) alkyl group, a (C₁-C₁₂) alkoxy group, and a (C₂-C₂₀) alkenyl group;
or R is selected from a (C₁-C₁₂) alkyl group and a (C₁-C₁₂) alkoxy group optionally substituted with one, two, or more OP(=O)(OH)₂;
or R is selected from a (C₁-C₁₂) alkyl group optionally substituted with one, two, or more -OP(O)(C₁-C₁₂ alkoxy)₂;
or R is selected from 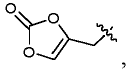 a (C₁-C₆) alkylC(=O)(C₂-C₆) alkenyl- group, and a 5-14 membered heteroaryl group, optionally substituted with one, two, or more (C₁-C₆) alkyl;
or R is selected from a (C₁-C₁₂) alkylC(=O)OCH(C₁-C₁₂ alkyl)- group, a C₃₋₁₂ cycloalkylC(=O)OCH(C₁-C₁₂ alkyl)- group, a (C₁-C₁₂) alkylC(=O)OCH₂- group, and a C₃₋₁₂ cycloalkylC(=O)OCH(C₁-C₁₂ alkyl)- group.

According to an embodiment of the present invention, the C₃₋₁₂ cycloalkyl group is preferably a C₃-₈ cycloalkyl group, selected from, for example, cyclopropyl, cyclobutyl, cyclopentyl, cyclohexyl, cycloheptyl, and cyclooctyl.

The 3-12 membered heterocyclyl group is preferably a 3-8 membered heterocyclyl group, selected from, for example, tetrahydrofuryl, tetrahydrothienyl, piperidinyl, dioxolonyl, pyrrolidinyl, imidazolidinyl, pyazolidinyl, pyrrolinyl, and 1,3-dioxazolyl.

The C₆₋₂₀ aryl group is preferably a C₆₋₁₀ aryl group, selected from, for example, phenyl.

The 5-14 membered heteroaryl group is preferably a 5-10 membered heteroaryl group, selected from indolyl, isoindolyl, pyridinyl, pyridazinyl, pyrimidinyl, pyrazinyl, triazinyl, thienyl, furyl, pyrrolyl, oxazolyl, thiazolyl, imidazolyl, pyrazolyl, isoxazolyl, isothiazolyl, oxadiazolyl, triazolyl, thiadiazolyl, and thia-4H-pyrazolyl.

According to an embodiment of the present invention, in some embodiments, Rb is attached to N in N-R₃ to form a ring structure 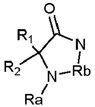 that is further preferably 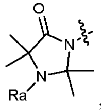 , and 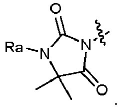 In this case, R₃ is absent, R₁, R₂ and Ra are as defined above in Formula I.

According to an embodiment of the present invention, R is selected from methyl, ethyl, n-propyl, iso-propyl, n-butyl, iso-butyl, t-butyl, n-pentyl, iso-pentyl, neo-pentyl, n-hexyl, ethenyl, 1-propenyl, 2-propenyl, 1-methylethenyl, 1-butenyl, 1-ethylethenyl, 1-methyl-2-propenyl, 2-butenyl, 3-butenyl, 2-methyl-1-propenyl, 2-methyl-2-propenyl, 1-pentenyl, 1-hexenyl, ethynyl, 1-propynyl, 2-propynyl, 1-butynyl, 1-methyl-2-propynyl, 3-butynyl, 1-pentynyl, 1-hexynyl, cyclopropyl, cyclobutyl, cyclopentyl, cyclohexyl, NH₂CH₂-, 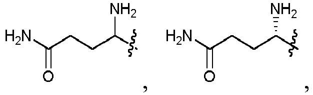 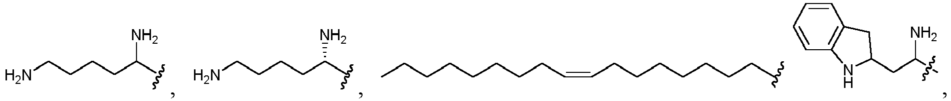 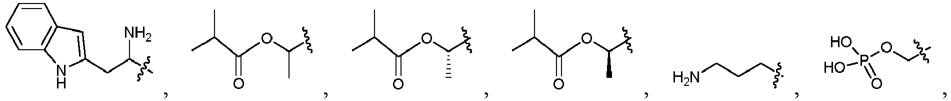 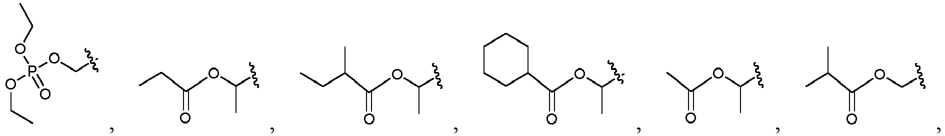 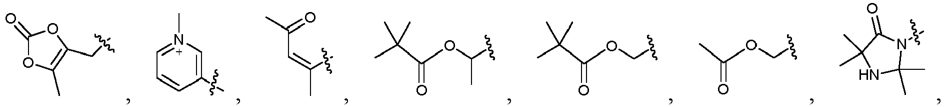 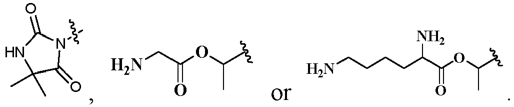 R is optionally further substituted with one, two or more Rc.

According to an embodiment of the present invention, the compound of Formula I is further preferably a compound of Formula II: 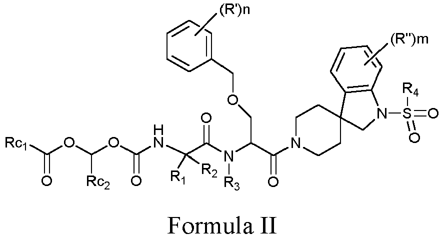
where Rc₁ and Rc₂ are the same or different, and each independently selected from H or Rc as defined above in Formula I; and
Rc, R', R", n, m, R₁, R₂, R₃, and R₄ as defined above in Formula I.

According to an embodiment of the present invention, the compound of Formula I is further preferably a compound of Formula III:

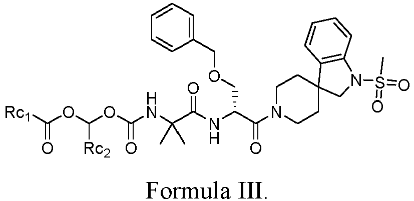

According to an embodiment of the present invention, in the structure of Formula III, Rc₁ and Rc₂ may be the same or different, and each independently selected from H or Rc as defined above in Formula I.

According to an embodiment of the present invention, the compound of Formula I is further preferably a compound of Formula IIIA or IIIB:

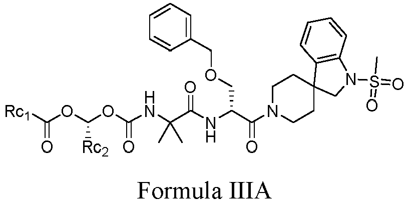

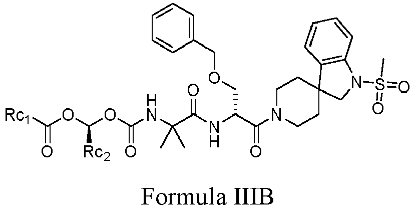

According to an embodiment of the present invention, in the structures of Formulas IIIA and IIIB, Rc₁ and Rc₂ may be the same or different, and each independently selected from H or Rc as defined above in Formula I.

According to an embodiment of the present invention, Rc₁ is selected from a C₁-C₁₂ alkyl group, a C₃-C₈ cycloalkyl group, and a C₁-C₁₂ alkyl group optionally substituted with one, two, or more NH₂; and Rc₂ is selected from H or a C₁-C₁₂ alkyl group.

According to an embodiment of the present invention, Rc₁ is selected from methyl, ethyl, iso-propyl, n-propyl, n-butyl, iso-butyl, t-butyl, aminomethyl, 1,5-diamino-n-pentyl, cyclopropyl, cyclobutyl, cyclopentyl or cyclohexyl.

According to an embodiment of the present invention, Rc₂ is selected from H or methyl.

According to an embodiment of the present invention, among the compound of Formula I and a racemate, a stereoisomer, a tautomer, an isotopically labeled compound, an N-oxide, a solvate, a polymorph, a metabolite, an ester, a prodrug or a pharmaceutically acceptable salt thereof, the exemplary and non-limiting examples of the compound of Formula I include 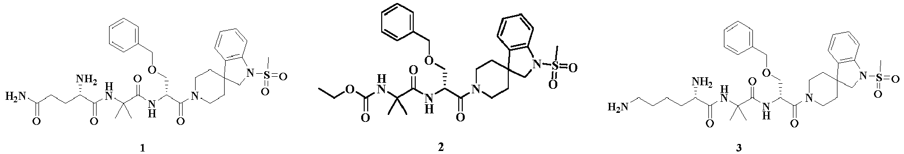 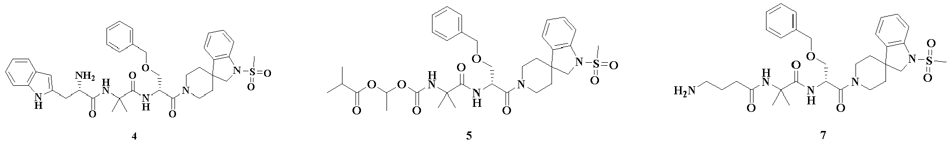 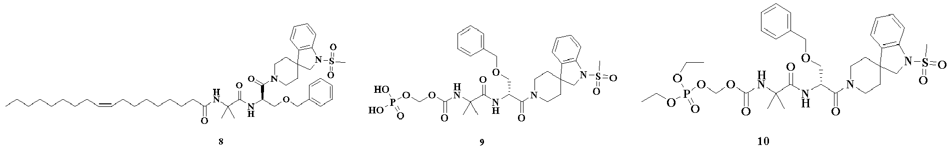 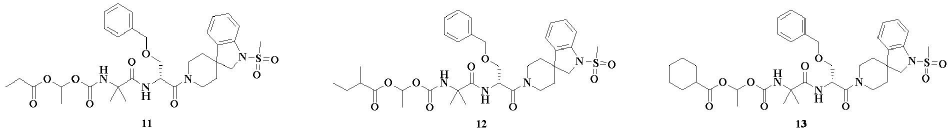 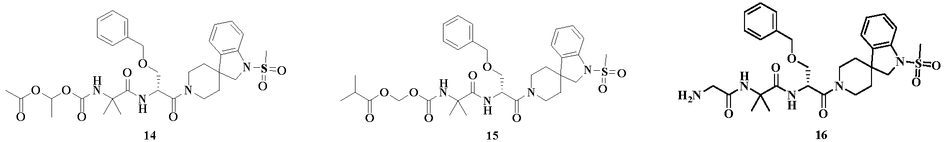 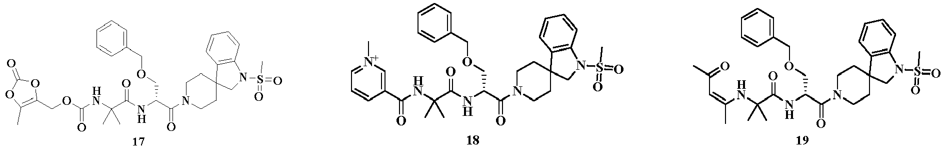 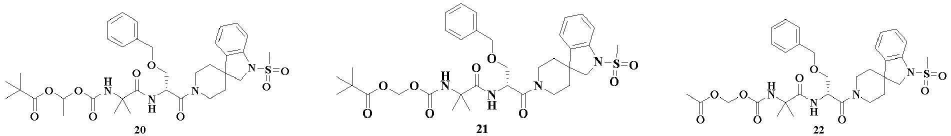 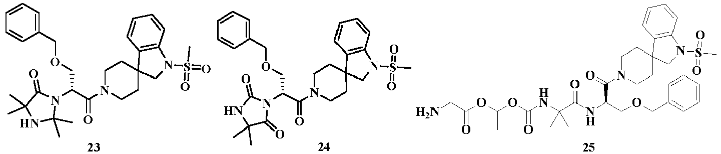 and

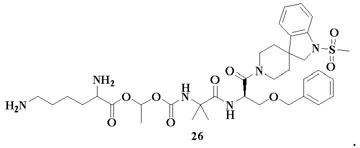

According to an embodiment of the present invention, the compound of Formula I may be further selected from the following structures:

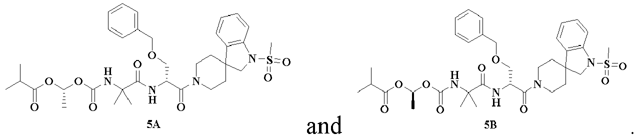

The present invention further provides a method for preparing the compound of Formula I, and a racemate, a stereoisomer, a tautomer, an isotopically labeled compound, an N-oxide, a solvate, a polymorph, a metabolite, an ester, a prodrug or a pharmaceutically acceptable salt thereof. The method includes the following steps:
reacting ibutamoren or a derivative thereof with a raw material capable of introducing the modifying group Ra and/or Rb in a suitable reagent under suitable conditions, to obtain an amino modified product; and optionally adding a protecting group and removing a protecting group under suitable conditions;

Alternatively, the method includes the following steps: reacting ibutamoren or a derivative thereof in the presence of potassium dihydrogen phosphate and acetone under suitable conditions.

Alternatively, the method includes the following steps: reacting ibutamoren or a derivative thereof in the presence of a chloroformate (or a substituted thiocarbonate) and a base under suitable conditions, where the base is selected from triethylamine, and the solvent used in the reaction is dichloromethane.

Alternatively, the method includes the following steps: undergoing a condensation reaction with an amino acid to form an amide in the presence of a condensing agent and a base, where the condensing agent is selected from 2-(7-azabenzotriazole)-N,N,N',N'-tetramethyluronium hexafluorophosphate and dicyclohexylcarbodiimide, and the base is selected from triethylamine.

The present invention further provides a pharmaceutical composition comprising the compound of Formula I according to the present invention, or a racemate, a stereoisomer, a tautomer, an isotopically labeled compound, an N-oxide, a solvate, a polymorph, a metabolite, an ester, a prodrug or a pharmaceutically acceptable salt thereof.

In some embodiments, the pharmaceutical composition of the present invention further comprises a therapeutically effective amount of the compound of Formula I according to the present invention or a racemate, a racemate, a stereoisomer, a tautomer, an isotopically labeled compound, an N-oxide, a solvate, a polymorph, a metabolite, an ester, a prodrug or a pharmaceutically acceptable salt thereof, and a pharmaceutically acceptable carrier.

The carrier in the pharmaceutical composition is "acceptable", compatible with (and preferably able to stabilize) the active ingredient in the composition and not harmful to the subject being treated. One or more solubilizers can be used as a pharmaceutical excipient to deliver the active compound.

The present invention further provides use of the compound of Formula I, or a racemate, a racemate, a stereoisomer, a tautomer, an isotopically labeled compound, an N-oxide, a solvate, a polymorph, a metabolite, an ester, a prodrug or a pharmaceutically acceptable salt thereof, or the pharmaceutical composition in the preparation of a GHSR agonist.

The present invention further provides use of the compound of Formula I, or a racemate, a racemate, a stereoisomer, a tautomer, an isotopically labeled compound, an N-oxide, a solvate, a polymorph, a metabolite, an ester, a prodrug or a pharmaceutically acceptable salt thereof, or the pharmaceutical composition in the preparation of drugs for diagnosing, preventing and/or treating growth hormone dependent diseases.

The present invention further provides a method for treating a disease or disorder, which comprises administering a therapeutically effective amount of at least one compound of the present invention to a patient in need of such treatment, alone or optionally in combination with another compound of the present invention and/or at least one therapeutic agent of other type.

According to an embodiment of the present invention, the disease or disorder is associated with growth hormone deficiency or growth hormone dependence. Preferably, the compound can promote the plasma level of growth hormone in human and animals after administration. This characteristic can be used to diagnose and treat physiological or medical symptoms characterized by deficiency of growth hormone secretion, for example, in the diagnosis of patients with growth hormone deficiency, children with slow growth and short stature due to growth hormone deficiency, and in the treatment of other diseases that can be improved by the physiological function of growth hormone.

In some embodiments, the compound of the present invention can be used to treat diseases requiring the stimulation to produce or secrete growth hormone, for example, humans suffering from natural growth hormone deficiency or animals used for food production. In the latter case, the stimulation of growth hormone will produce larger and more productive animals. The compound of the present invention is used to treat humans and animals to increase the level of growth hormone secretion.

In some embodiments, the compound is used as a GHSR agonist, for use in, without limitation, energy balance and food intake regulation; treatment of fat formation and obesity and reduction of body weight; treatment of cachexia; improvement of gastrointestinal motility, and treatment of gastroparesis and diabetic gastroparesis, and postoperative intestinal obstruction; the increase of muscle mass and skin thickness, the decrease of fatty substances and the slight increase of bone mineral density in the elderly patients; treatment of burns, AIDS and cancers; and healing of wounds and bones.

The compound of the present invention can be used in combination with an additional therapeutic agent.

The present invention further provide a method for preventing and/or treating growth hormone-dependent diseases, which comprises administering a therapeutically effective amount of a first and a second therapeutic agent to a patient in need thereof, where the first therapeutic agent is a compound of the present invention. In some embodiments, the present invention provides a combined preparation of the compound of the present invention and an additional therapeutic agent for simultaneous, separate or sequential administration in treatment.

### Definitions of terms

Unless otherwise specified, the definitions of groups and terms in the specification and claims of the present application, including the definitions as examples, exemplary definitions, preferred definitions, definitions recorded in tables, definitions of specific compounds in Examples, etc., can be arbitrarily combined with each other. Such group definitions and compound structures obtained after combination should fall in the scope recorded in the specification of the present application.

The term "optional" (or "optionally") in the definition for the general formula of the present application means the substitution with zero, one or more substituents. For example, "optionally substituted with one, two or more R" means no substitution (unsubstitution) with R or substitution with one, two or more R.

When the numerical range recorded in the specification and claims of the present application is defined as an integer, or is often an integer according to the conventional understanding in the field, the numerical range should be understood as recording two endpoints of the range and every integer within the range. For example, when "0-12" indicates the number of carbon, it should be understood that every integer of 0, 1, 2, 3, 4, 5, 6, 7, 8, 9, 10, 11 and 12 is recorded. "More in more than one" means two or more, and "more in two or more" means three or more.

The term "halo" refers to F, Cl, Br and I. In other words, F, Cl, Br and I can be described as "halo" in the specification.

The term "aliphatic hydrocarbyl" includes saturated or unsaturated, linear or branched, or cyclic hydrocarbyl groups. The aliphatic hydrocarbyl group can be selected from alkyl, alkenyl, alkynyl, and so on, and the aliphatic hydrocarbyl group may have 1-20 (including 1, 2, 3, 4, 5, 6, 7, 8, 9, 10, 11, 12, 13, 14, 15, 16, 17, 18, 19, and 20), preferably 1-12, optionally 1-10, and further preferably 1-6 carbon atoms. Specific examples may include, but is not limited to, methyl, ethyl, n-propyl, iso-propyl, n-butyl, iso-butyl, t-butyl, n-pentyl, iso-pentyl, neo-pentyl, n-hexyl, ethenyl, 1-propenyl, 2-propenyl, 1-methylethenyl, 1-butenyl, 1-ethylethenyl, 1-methyl-2-propenyl, 2-butenyl, 3-butenyl, 2-methyl-1-propenyl, 2-methyl-2-propenyl, 1-pentenyl, 1-hexenyl, ethynyl, 1-propynyl, 2-propynyl, 1-butynyl, 1-methyl-2-propynyl, 3-butynyl, 1-pentynyl, 1-hexynyl, cyclopropyl, cyclobutyl, cyclopentyl, and cyclohexyl. The "aliphatic hydrocarbyl" moiety contained in other groups has the meanings.

The term "alkyl" should be understood to represent a linear or branched saturated monovalent hydrocarbyl group, preferably, a linear or branched saturated monovalent hydrocarbyl group containing 1-20 carbon atoms. For example, "C₁₋₁₀ alkyl" represents a linear or branched alkyl group having 1, 2, 3, 4, 5, 6, 7, 8, 9 or 10 carbon atoms, "C₁₋₈ alkyl" represents a linear or branched alkyl group having 1, 2, 3, 4, 5, 6, 7 or 8 carbon atoms, and "C₁₋₆ alkyl" represents a linear or branched alkyl group having 1, 2, 3, 4, 5 or 6 carbon atoms. The alkyl is, for example, methyl, ethyl, propyl, butyl, pentyl, hexyl, iso-propyl, iso-butyl, s-butyl, t-butyl, iso-pentyl, 2-methylbutyl, 1-methylbutyl, 1-ethylpropyl, 1,2-dimethylpropyl, neo-pentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or 1,2-dimethylbutyl, or an isomer thereof.

The term "alkenyl" should be understood to represent a linear or branched monovalent hydrocarbyl group containing one or more double bonds and having 2-20 carbon atoms. "C₂₋₁₀ alkenyl" is preferred. "C₂₋₁₀ alkenyl" should be understood to represent a linear or branched monovalent hydrocarbyl group containing one or more double bonds and having 2, 3, 4, 5, 6, 7, 8, 9 or 10 carbon atoms. "C₂₋₈ alkenyl" is further preferred, which contains one or more double bonds and has 2, 3, 4, 5, 6, 7 or 8 carbon atoms, for example, 2, 3, 4, 5 or 6carbon atoms (that is, C₂₋₆ alkenyl), 2 or 3 carbon atoms (that is, C₂₋₃ alkenyl). It is to be understood that when the alkenyl group containing more than one double bond, the double bonds can be separated from each other or conjugated. The alkenyl group includes, for example, ethenyl, allyl, (E)-2-methylethenyl, (Z)-2-methylethenyl, (E)-but-2-enyl, (Z)-but-2-enyl, (E)-but-1-enyl, (Z)-but-1-enyl, pent-4-enyl, (E)-pent-3-enyl, (Z)-pent-3-enyl, (E)-pent-2-enyl, (Z)-pent-2-enyl, (E)-pent-1-enyl, (Z)-pent-1-enyl, hex-5-enyl, (E)-hex-4-enyl, (Z)-hex-4-enyl, (E)-hex-3-enyl, (Z)-hex-3-enyl, (E)-hex-2-enyl, (Z)-hex-2-enyl, (E)-hex-1-enyl, (Z)-hex-1-enyl, isopropenyl, 2-methylprop-2-enyl, 1-methylprop-2-enyl, 2-methylprop-1-enyl, (E)-1-methylprop-1-enyl, (Z)-1-methylprop-1-enyl, 3-methylbut-3-enyl, 2-methylbut-3-enyl, 1-methylbut-3-enyl, 3-methylbut-2-enyl, (E)-2-methylbut-2-enyl, (Z)-2-methylbut-2-enyl, (E)-1-methylbut-2-enyl, (Z)-1-methylbut-2-enyl, (E)-3-methylbut-1-enyl, (Z)-3-methylbut-1-enyl, (E)-2-methylbut-1-enyl, (Z)-2-methylbut-1-enyl, (E)-1-methylbut-1-enyl, (Z)-1-methylbut-1-enyl, 1,1-dimethylprop-2-enyl, 1-ethylprop-1-enyl, 1-propylethenyl, and 1-isopropylethenyl.

The term "alkynyl" should be understood to represent a linear or branched monovalent hydrocarbyl group containing one or more triple bonds and having 2-20 carbon atoms. "C₂₋₁₀ alkynyl" is preferred. The term "C₂₋₁₀ alkynyl" should be understood to preferably represent a linear or branched monovalent hydrocarbyl group, containing one or more triple bonds and having 2, 3, 4, 5, 6, 7, 8, 9 or 10 carbon atoms, for example, 2, 3, 4, 5, 6, 7 or 8 carbon atoms (that is, "C₂₋₈alkynyl"), 2, 3, 4, 5 or 6 carbon atoms (that is, "C₂₋₆alkynyl"), and 2 or 3carbon atoms ("C₂₋₃alkynyl"). The alkynyl is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-iso-propylprop-2-ynyl, 2,2-dimethylbut-3-ynyl, 1,1-dimethylbut-3-ynyl, 1,1-dimethylbut-2-ynyl or 3,3-dimethylbut-1-ynyl. Particularly, the alkynyl group is ethynyl, prop-1-ynyl or prop-2-ynyl.

The term "aliphatic hydrocarbylene" refers to a divalent aliphatic hydrocarbon chain. It can be understood by those skilled in the art that when the aliphatic hydrocarbyl group occurs at a substitution position forming a divalent group in the structural formula, it is equivalent to the aliphatic hydrocarbylene. Therefore, in the present application, the aliphatic hydrocarbylene group can also interpreted as for the "aliphatic hydrocarbyl" group. For example, the aliphatic hydrocarbylene can be selected from alkylene, alkenylene, alkynylene, and so on. The aliphatic hydrocarbylene can have 1-20 (including 1, 2, 3, 4, 5, 6, 7, 8, 9, 10, 11, 12, 13, 14, 15, 16, 17, 18, 19, and 20), preferably 1-12, optionally 1-10, and further preferably 1-6 carbon atoms.

The term "C₃₋₁₂ cycloalkyl" should be understood to represent a saturated or unsaturated monovalent monocyclic or bicyclic ring, having 3-12 carbon atoms. C₃₋₈ cycloalkyl is preferred, and C₃₋₆ cycloalkyl is further preferred. For example, the C₃₋₈ cycloalkyl should be understood to represent a saturated or unsaturated monovalent monocyclic or bicyclic ring, having 3, 4, 5, 6, 7 or 8 carbon atoms. The C₃₋₁₂ cycloalkyl can be a monocyclic hydrocarbyl group, for example, cyclopropyl, cyclobutyl, cyclopentyl, cyclohexyl, cycloheptyl, cyclooctyl, cyclononyl or cyclodecyl, or a bicyclic hydrocarbyl group, for example, tetrahydronaphthalene or decahydronaphthalene.

The term "3-12 membered heterocyclyl" means a saturated or unsaturated monovalent monocyclic or bicyclic ring, containing 1-5 (preferably 1-3) heteroatoms independently selected from N, O and S, where the heteroatom-containing group is not aromatic. 3-10 membered heterocyclyl group and 3-8 membered heterocyclyl group are preferred. The heterocyclyl group can be connected to the rest of a molecule through any one of the carbon atoms or nitrogen atom (if present). Particularly, the heterocyclyl group can include, but is not limited to, a 4-membered ring, such as azetidinyl and oxetanyl; a 5-membered ring, such as tetrahydrofuryl, tetrahydrothienyl, dioxolonyl, pyrrolidinyl, imidazolidinyl, pyazolidinyl, pyrrolinyl, and 1,3-dioxazolyl; or a 6-membered ring, such as tetrahydropyranyl, piperidinyl, morpholinyl, dithianyl, thiomorpholinyl, piperazinyl or trithianyl; or a 7-membered ring, such as diazepanyl. Optionally, the heterocyclyl group may be benzo-fused. The heterocyclyl group may be bicyclic, for example, but not limited to, a 5,5-membered ring, such as hexahydrocyclopenta[c]pyrrol-2(1H)-yl, or a 5,6-membered ring, such as hexahydropyrrolo[1,2-a]pyrazin-2(1*H*)-yl. The ring containing nitrogen atom can be partially unsaturated, that is, it can contain one or more double bonds, for example, but not limited to, 2,5-dihydro-1H-pyrrolyl, 4H-[1,3,4]thiadiazinyl, 4,5-dihydrooxazolyl or 4H-[1,4]thiazinyl; or it can be benzo-fused, for example, but not limited to, dihydroisoquinolyl, 2,3-dihydrobenzofuryl, 3,4-dihydro-2H-1-benzopyranyl (chromanyl), 2,3-dihydrobenzo[b][1,4]dioxanyl. The 3-12 membered heterocyclyl group may also be selected from, for example,

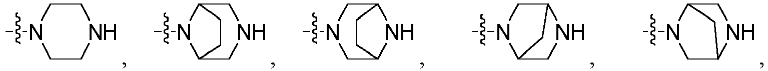

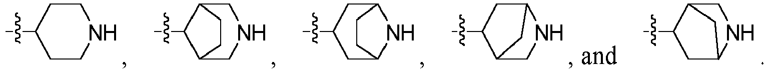

The term "C₆₋₂₀ aryl" should be understood to preferably represent a monovalent aromatic or partially aromatic monocyclic, bicyclic or tricyclic hydrocarbon ring having 6-20 carbon atoms. The "C₆₋₁₄ aryl" group or "C₆₋₁₀ aryl" group is preferred. The term C₆₋₂₀ aryl should be understood to preferably represent a monovalent aromatic or partially aromatic monocyclic, bicyclic or tricyclic hydrocarbon ring having 6, 7, 8, 9, 10, 11, 12, 13, 14, 15, 16, 17, 18, 19, or 20 carbon atoms, particularly, a ring having 6 carbon atoms ("C₆ aryl"), for example, phenyl; biphenylyl; a ring having 9 carbon atoms ("C₉ aryl"), for example, indanyl or indenyl; a ring having 10 carbon atoms ("C₁₀ aryl"), for example, tetrahydronaphthyl, dihydronaphthyl or naphthyl; a ring having 13 carbon atoms ("C₁₃ aryl"), for example, fluorenyl; or a ring having 14 carbon atoms ("C₁₄ aryl"), for example, anthryl.

The term "5-14 membered heteroaryl" should be understood to include a monovalent monocyclic, bicyclic or tricyclic aromatic ring system that has 5, 6, 7, 8, 9, 10, 11, 12, 13 or 14 ring atoms, particularly 5, 6, 9 or 10 carbon atoms, and contains 1-5 and preferably 1-3 heteroatoms independently selected from N, O and S; and may be benzo-fused at each occurrence. Particularly, the heteroaryl is selected from thienyl, furyl, pyrrolyl, oxazolyl, thiazolyl, imidazolyl, pyrazolyl, isoxazolyl, isothiazolyl, oxadiazolyl, triazolyl, thiadiazolyl, thia-4H-pyrazolyl, and their benzo-fused derivatives, for example, benzofuryl, benzothienyl, benzooxazolyl, benzoisoxazolyl, benzoimidazolyl, benzotriazolyl, indazolyl, indolyl, isoindolyl, and so on; or pyridinyl, pyridazinyl, pyrimidinyl, pyrazinyl, triazinyl, and their benzo-fused derivatives, for example, quinolyl, quinazolinyl, isoquinolyl, and so on; or azocinyl, indolizinyl, purinyl and their benzo-fused derivatives; or cinnolinyl, phthalazinyl, quinazolinyl, quinoxalinyl, naphthyridinyl, pteridinyl, carbazolyl, acridinyl, phenazinyl, phenothiazinyl, phenoxazinyl and so on.

The term "alkoxy" refers to -O-(alkyl) and -O-(unsubstituted cycloalkyl), where the alkyl is as defined above. Non-limiting examples of the alkoxy include methoxy, ethoxy, propoxy, butoxy, cyclopropoxy, cyclobutoxy, cyclopentoxy, and cyclohexyloxy. The alkoxy may be optionally substituted or unsubstituted. When substituted, the substituent is preferably one or more groups independently selected from alkyl, alkenyl, alkynyl, alkoxy, alkylthio, alkylamino, halo, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate ester group.

The term "oxo" refers to a substitution of oxygen (=O) formed when a carbon atom, nitrogen atom or sulfur atom in a substituent is oxidized.

"DCM" refers to dichloromethane. "MeOH" refers to methanol. "DMF" refers to N,N-dimethylformamide. "DIPEA" refers to N,N-diiso-propylethyl amine. "HATU" refers to 2-(7-azabenzotriazol)-N,N,N',N'-tetramethyluronium hexafluorophosphate.

Unless otherwise specified, the heterocyclyl or heteroaryl includes all possible isomeric forms, such as positional isomers. Therefore, some illustrative and non-limiting examples of the pyridinyl or pyridinylene group pyridin-2-yl, pyridin-2-ylene, pyridin-3-yl, pyridin-3-ylene, pyridin-4-yl and pyridin-4-ylene; some illustrative and non-limiting examples of thienyl or thienylene include thien-2-yl, thien-2-ylene, thien-3-yl, and thien-3-ylene.

According to the molecular structure, the compound of the present invention can be chiral, so there may be various enantiomeric forms. Therefore, these compounds can exist in racemate form or optically active form. The compound of the present invention or an intermediate thereof can be separated into enantiomeric compounds by chemical or physical methods known to those skilled in the art, or used in synthesis in the form of enantiomeric compounds. In the case of racemic amines, a diastereomer is prepared from a mixture by reacting with an optically active resolution reagents. Examples of suitable resolution reagents include optically active acids, for example, tartaric acid in R and S configuration, diacetyl tartaric acid, dibenzoyl tartaric acid, mandelic acid, malic acid, lactic acid, suitable N- protected amino acid (for example, N-benzoyl proline or N-phenylsulfonyl proline) or various optically active camphorsulfonic acids. With the aid of an optically active resolution reagent (for example, dinitrobenzoylphenylglycine, cellulose triacetate or derivatives of other carbohydrates or chirally derivatizing methacrylate polymers, immobilized on silica gel), enantiomers can also be advantageously resolved by chromatography. For example, A suitable eluent for this purpose is an aqueous or alcoholic solvent mixture, for example, hexane/isopropanol/acetonitrile.

Those skilled in the art will understand that not all nitrogen-containing heterocyclic rings can be oxidized to form an N-oxide because available lone pair electrons are required by nitrogen to be oxidized into an N-oxide. Those skilled in the art can identify nitrogen-containing heterocyclic rings that can form an N-oxide. Those skilled in the art will also recognize that tertiary amines can form an N- oxide. A synthesis method for preparing N-oxides of heterocyclic rings and tertiary amines is well known to those skilled in the art, and includes oxidizing heterocyclic rings and tertiary amines with peroxyacids such as peracetic acid and m-chloroperoxybenzoic acid (MCPBA), hydrogen peroxide, alkyl hydroperoxides such as t-butyl hydroperoxide, sodium perborate, and dioxiranes such as dimethyl dioxirane. These methods for preparing N-oxides are widely described and reviewed in the literatures.

The pharmaceutically acceptable salt can be, for example, an acid addition salt of the compound of the present invention that has a nitrogen atom in the chain or ring and has sufficient alkalinity.

In addition, the basic nitrogen-containing group can be quaternized with the following reagents: a lower alkyl halide, for example, methyl, ethyl, propyl and butyl chloride, bromide and iodide; a dialkyl sulfate, for example, dimethyl sulfate, diethyl sulfate, dibutyl sulfate and amyl sulfate; a long-chain halide, for example, decyl, lauryl, myristyl and stearyl chloride, bromide and iodide; and an aralkyl halide such as benzyl and phenyl ethyl bromide, etc. Examples of pharmaceutically acceptable salts include hydrochloride, sulfate, nitrate, bisulfate, hydrobromide, acetate, oxalate, citrate, methanesulfonate, formate or meglumine salt.

Since the compound of the present invention can have multiple salt-forming sites, the pharmaceutically acceptable salt includes not only the salt formed at 1 salt-forming site, but also the salt formed at 2, 3 or all the salt-forming sites of the compound of the present invention. Therefore, in the pharmaceutically acceptable salt, the molar ratio of the compound of Formula (I) to the anion of an acid or cation of a base required for salt formation can vary in a wide range, for example, 4:1 to 1:4, for example, 3:1, 2:1, 1:1, 1:2, and 1:3, etc.

According to the positions and properties of different substituents, the compound of the present invention may further have one or more asymmetric centers. The asymmetric carbon atoms can exist in (R) or (5) configuration. When there is only one asymmetric center, a racemic mixture is produced, and when there are multiple asymmetric centers, a diastereomeric mixture is obtained. In some cases, asymmetry may occur due to the hindrance of rotation around a specific bond which connects two substituted aromatic rings of a specific compound. Moreover, the substituents can also exist in the form of cis or trans isomer.

The compound of the present invention also include all possible stereoisomers thereof, and is in the form of a single stereoisomer or any mixture of the stereoisomers (for example, R-isomer or S-isomer, or E-isomer or Z-isomer) at any ratio. The separation of a single stereoisomer (for example, single enantiomer or single diastereomer) of the compound of the present invention can be achieved by any suitable method in the prior art (for example, chromatography, particularly for example, chiral chromatography).

The term "tautomer" refers to a functional isomer produced by the rapid movement of an atom between two positions in a molecule. The compound of the present invention can show tautomerism. There may be two or more tautomeric compounds that can be converted into each other. Proton shift tautomers originate from the migration of hydrogen atoms covalently bonded between two atoms. Tautomers generally exist in equilibrium. When attempts are made to separate a single tautomer, a mixture is usually produced, having physical and chemical properties that are consistent with the mixture of the compounds. The position of equilibrium depends on the intramolecular chemical characteristics. For example, in many aliphatic aldehydes and ketones such as acetaldehyde, ketone type is dominant; and in phenols, enol type is dominant. The present invention includes all tautomeric forms of the compound.

In the present invention, the compound involved also include an isotopically labeled compound, which is the same as the compound of Formula I, except that one or more atoms are replaced by an atom whose atomic mass or mass number is different from that of an atom that usually occurs naturally. Exemplary isotopes that can be introduced into the compound of the present invention include isotopes of H, C, N, O, P, S, F and Cl, such as ²H, ³H, ¹³C, ¹¹C, ¹⁴C, ¹⁵N, ¹⁸O, ¹⁷O, ³²P, ³⁵S, ¹⁸F and ³⁶Cl. The compound of the present invention containing the aforementioned isotopes and/or other isotopes of other atoms and a prodrug, or a pharmaceutically acceptable salt of the compound or prodrug are covered in the scope of the present invention. Some isotopically labeled compounds of the present invention, for example, compounds having radioactive isotopes (such as ³H and ¹⁴C) can be used in the distribution determination of drugs and/or substrates in tissues. The isotopes tritium (that is, ³H) and carbon 14 (that is, ¹⁴C) are particularly preferred because of their easy preparation and detectability. Furthermore, the substitution with a heavier isotope (such as deuterium, that is, ²H) can provide some therapeutic advantages due to greater metabolic stability (for example, increased in vivo half-life or reduced dosage requirement), and is thus preferred in some cases. The compound of the present invention as claimed in the claims can be particularly defined to be substituted with deuterium or tritium. In addition, in case that hydrogen in a substituent is not explicitly indicated to be deuterium or tritium, it does not mean that deuterium or tritium is excluded, but deuterium or tritium can be contained as well.

The term "effective amount" or "therapeutically effective amount" refers to an amount of the compound of the present invention that is sufficient to achieve the intended use (including, but not limited to, the diagnosis, prevention or treatment of diseases as defined above). The therapeutically effective amount varies depending on the following factors: the expected use (in vitro or in vivo), or the subjects and diseases and disorders treated, such as the weight and age of the subjects, the severity of diseases and disorders, and the mode of administration, etc., which can be easily determined by those skilled in the art. The specific dosage will vary depending on the following factors: the specific compound selected, the administration scheme, whether the compound is combined with other compounds, the dosing regimen, the tissue to which the compound is administered, and the physical delivery system carrying the compound.

The term "solvates" are forms of the compound of the present invention, which are complexes formed by coordination of the compound of the present invention in the state of solid or liquid with a solvent molecule. Hydrate is a particulate form of the solvate, in which coordination is carried out with water. In the present invention, the preferred solvate is hydrate. Furthermore, the pharmaceutically acceptable solvate (hydrate) of the compound of general Formula I of the present invention refers to an eutectic and inclusion compound formed by Compound I with stoichiometric one or more molecules of water or other solvents. Solvents useful for the solvates include, but are not limited to, water, methanol, ethanol, ethylene glycol and acetic acid.

The term "prodrug" represents a compound that is transformed into the compound of general formula above or a specific compound in vivo. Such conversion is affected by the hydrolysis of the prodrug in blood or the enzymatic conversion in blood or tissues into the parent structure. The prodrug of the present invention can be an ester. In the present invention, esters useful as a prodrug include phenyl esters, aliphatic esters, acyloxymethyl esters, carbonates, carbamates and amino acid esters. For example, one compound of the present invention contains a hydroxyl/carboxyl group, then it can be acylated to obtain a compound as a prodrug. Other prodrug forms include phosphates, for example, obtained by phosphorylation of a hydroxyl group on the parent compounds.

### Beneficial effects

The compound provided in the present invention has good GHSR agonist activity. The compound of the present invention not only has good biological activity and good safety, but also improves the transmembrane activity and drug bioavailability. Regarding the problems of poor injection compliance in the treatment of diseases with recombinant human growth hormone and lack of specific drugs for clinical diagnosis at present, the compound of the present invention improves the clinical compliance, and is expected to be used in place of recombinant human growth hormone in many cases, in the diagnosis, prevention or treatment of diseases in the area of growth retardation.

### BRIEF DESCRIPTION OF THE DRAWINGS

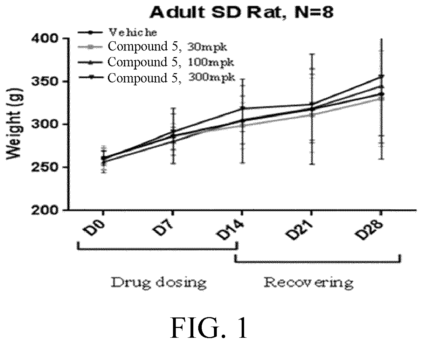

FIG. 1 shows weight change of rats administered with Compound 5 for consecutive 14 days.

### DETAILED DESCRIPTION

Hereinafter, the technical solutions of the present invention will be described in further detail with reference to specific examples. It should be understood that the following examples only illustratively describe and explain the present invention, and should not be construed as limiting the protection scope of the present invention. All technologies accomplished based on the above disclosure of the present invention are contemplated in the scope of the present invention.

The structure of the compound of the present invention is determined by nuclear magnetic resonance (NMR) spectroscopy or/and liquid chromatography-mass spectrometry (LC-MS). The chemical shift (δ) in NMR spectroscopy is given in parts per million (ppm). The NMR spectrum is determined by Bruker AVANCE-400 Nuclear Magnetic Resonance Spectrometer, the solvents for determination include deuterated dimethyl sulfoxide (DMSO-*d ₆*), deuterated methanol (CD₃OD) and deuterated chloroform (CDCl₃), and the internal standard is tetramethylsilane (TMS).

Agilent 1200 Infinity Series mass spectrometer is used for LC-MS. The HPLC spectrum is determined by Agilent 1200DAD high performance liquid chromatographic instrument (Sunfire C18 150 × 4.6 mm column) and Waters 2695-2996 high performance liquid chromatographic instrument (Gimini C18 150 × 4.6mm column).

The silica gel plate used for thin layer chromatography is Yantai Huanghai HSGF254 or Qingdao GF254 silica gel plate. The specification of the silica gel plate used for TLC is 0.15 mm- 0.20 mm. The specification for separation and purification by thin layer chromatography is 0.4 mm- 0.5 mm. In column chromatography, Yantai Huanghai silica gel of 200- 300 meshes is used as a carrier.

Unless otherwise specified, the raw materials and reagents used in the following examples are all commercially available, and or can be prepared by known methods. Unless otherwise particularly specified, all the reactions in the present invention are carried out with continuous magnetic stirring under dry nitrogen or argon atmosphere, the solvent is dry solvent, and the reaction temperatures is in degree Celsius.

### Example 1

### (2S)-2-amino-N-(1-{[((2R)-3-(benzoxy)-1- { 1-methansulfonyl-1,2-dihydrospiro[indol-3,4-piperidin] -1-yl } -1-oxopropan-2-yl]carbamoyl} -1-methylethyl)glutaramide

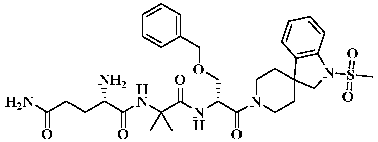

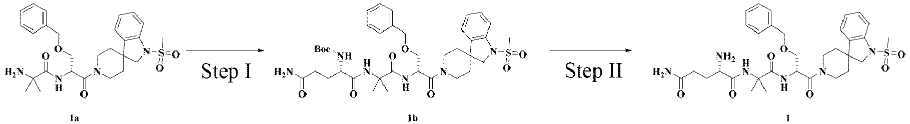

### Step I

### T-butyl N-[(1S)-1-[[1-{[(2R)-3-(benzoxy)-1- {1-methansulfonyl-1,2-dihydrospiro[indol-3,4-piperidin]- 1-yl} -1-oxopropan-2-yl]carbamoyl} -1-methylethyl)carbamoyl] -3-carbamoylpropyl]carbamate

Ibutamoren **1a** (350 mg, 0.66 mmol) was dissolved in dichloromethane (20 mL), and then N,N-diiso-propylethyl amine (255 mg, 1.98 mmol), 2-(7-azabenzotriazole)-N,N,N',N'-tetramethyluronium hexafluorophosphate (376 mg, 0.99 mmol) and (2S)-2-{[ (t-butoxy)carbonyl]amino} -4-carbamoylbutyric acid (195 mg, 0.79 mmol) were added to the mixture. The reaction was stirred at 25°C for 1 hr. After the reaction was completed, the solvent was removed by rotary evaporation to obtain a crude product. The crude product was separated and purified by running through a reversed-phase column (acetonitrile:water = 1: 1) to obtain t-butyl N-[(1S)-1-[(1-{[(2R)-3-(benzoxy)-1- {1-methansulfonyl-1,2-dihydrospiro[indol-3,4'-piperidin] -1'-yl} -1-oxopropan-2-yl]carbamoyl} -1-methylethyl)carbamoyl] -3-carbamoylpropyl]carbamate **1b** (300 mg, white solid). Yield: 54%.

MS m/z (ESI): 757.2[M+1]⁺.

### Step II

### Preparation of (2S)-2-amino-N-(1-{ [((2R)-3-(benzoxy)-1- {1-methansulfonyl-1,2-dihydrospiro[indol-3,4-piperidin] -1-yl } -1-oxopropan-2-yl]carbamoyl} -1-methylethyl)glutaramide

T-butyl N-[(1S)-1-[[1-{[(2R)-3-(benzoxy)-1- {1-methansulfonyl-1,2-dihydrospiro[indol-3,4-piperidin]- 1-yl} -1-oxopropan-2-yl]carbamoyl} -1-methylethyl)carbamoyl] -3-carbamoylpropyl]carbamate **1b** (300 mg, 0.4 mmol) was dissolved in dichloromethane (10 mL), and then trifluoroacetic acid (3 mL) was added. The reaction was stirred at room temperature for 1 hr. After the reaction was completed, the solvent was removed by rotary evaporation to obtain a crude product. The crude product was separated and purified by running through a reversed-phase column (acetonitrile:water = 2:3) to obtain the product (2S)-2-amino-N-(1-{[((2R)-3-(benzoxy)-1- {1-methansulfonyl-1,2-dihydrospiro[indol-3,4-piperidin] -1-yl } -1-oxopropan-2-yl]carbamoyl} -1-methylethyl)glutaramide **1** (179 mg, pale yellow solid). Yield: 68%.

MS m/z (ESI): 657.2[M+1]⁺.

HPLC: 99.28% (214 nm), 100% (254 nm).

¹H NMR (400 MHz, MeOD) *δ* 7.67 (dd, *J* = 24.4, 8.0 Hz, 1H), 7.36-7.25 (m, 7H), 7.25-7.18 (m, 1H), 7.06-6.96 (m, 1H), 6.76 (d, J = 7.4 Hz, 1H), 5.20-5.16 (m , 1H), 4.56 (s, 1H), 4.54-4.44 (m, 2H), 4.10-4.04 (m, 1H), 3.98-3.84 (m, 3H), 3.76-3.65 (m, 2H), 3.28-3.09 (m, 1H), 2.96 (d, *J* = 7.2 Hz, 3H), 2.90-2.80 (m, 1H), 2.47 (t, *J* = 7.4 Hz, 2H), 2.15-2.09 (m, 2H), 1.99-1.85 (m, 1H), 1.74-1.66 (m, 3H), 1.51 (t, *J* = 6.0 Hz, 6H).

### Example 2

### Ethyl N-(1-{[(2R)-3-(benzoxy)-1-{1-methansulfonyl-1,2-dihydrospiro[indol-3,4-piperidin]-1-yl]-1-oxopropan-2-yl] carbamoyl}-1-methylethyl)carbamate

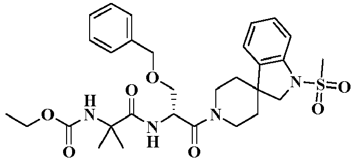

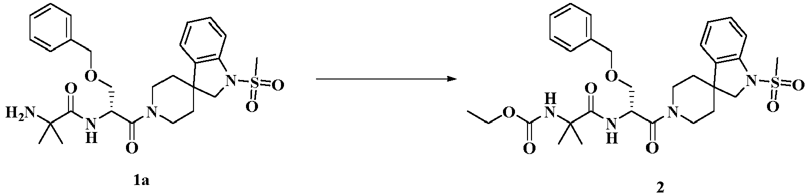

### Step I

### Preparation of ethyl N-(1-{ [(2R)-3-(benzoxy)-1-{1-methansulfonyl-1,2-dihydrospiro[indol-3,4-piperidin]-1-yl]-1-oxopropan-2-yl] carbamoyl}-1-methylethyl)carbamate

Ibutamoren **1a** (300 mg dissolved in dichloromethane (20 mL)) was added to saturated sodium carbonate solution (4 mL) and ethyl chloroformate (75 mg). The reaction was stirred at room temperature for 1 hr. After the reaction was completed, the reaction solution was concentrated under reduced pressure, and the crude product was purified by running through a reversed-phase column (acetonitrile:water = 1:5) to obtain ethyl N-(1-{[(2R)-3-(benzoxy)-1-{1-methansulfonyl-1,2-dihydrospiro[indol-3,4-piperidin]-1-yl]-1-oxopropan-2-yl] carbamoyl}-1-methylethyl)carbamate **2** (210 mg, white solid). Yield: 62%.

MS m/z (ESI): 601.4[M+1]⁺.

HPLC: 100% (214 nm), 100% (254 nm).

¹H NMR (400 MHz, CDCl₃) *δ* 7.41-7.31 (m, 4H), 7.30-7.19 (m, 1H), 7.24-7.19 (m, 1H), 7.12-7.04 (m, 1H), 6.98-6.94 (m, 1H), 6.61 (d, J=7.2 Hz, 1H), 5.21-5.12 (m, 2H), 4.68-4.44 (m, 3H), 4.08 (dd, J=14.2, 7.2 Hz, 2H), 4.02-3.98 (m,1H), 3.89-3.81 (m, 1H), 3.77 (d, J=10.4 Hz, 1H), 3.73-3.68 (m, 1H), 3.62-3.58 (m, 1H), 3.18-3.00 (m, 1H), 2.90 (d, J=4.4 Hz, 2H), 2.85-2.67 (m, 1H), 1.94-1.83 (m, 1H), 1.80-1.61 (m, 3H), 1.52 (d, *J*=8.4 Hz, 6 H), 1.23 (t, *J*=7.2 Hz, 3H).

### Example 3

### (2S)-2,6-diamino-N-(1- f [(2R)-3-(benzoxy)-1- f 1-methansulfonyl-1,2-dihydrospiro[indol-3,4-piperidin]-1-yl]-1-oxopropan-2-yl]carbamoyl}-1-methylethyl)caproamide

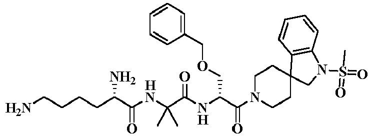

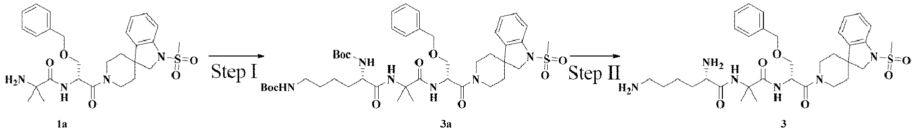

### Step I

### Preparation of t-butyl N-[(SS)-5-[(1-{ [(2R)-3-(benzoxy)-1-{ 1-methansulfonyl-1,2-dihydrospiro[indol-3,4-piperidin]-1-yl]-1-oxopropan-2-yl]carbamoyl}-1-methylethyl)carbamoyl]-5-{[(t-butoxy)carbonyl]amino}pentyl]carbamate

Ibutamoren **1a** (200 mg, 0.38 mmol) was dissolved in N,N-dimethylformamide (3 mL), N,N-diiso-propylethyl amine (147 mg, 1.14 mmol) and 2-(7-azabenzotriazole)-N,N,N',N'-tetramethyluronium hexafluorophosphate (216 mg, 0.57 mmol) were added, and finally (2S)-2,6-bis({[(t-butoxy)carbonyl]amino})capronic acid (158 mg, 0.46 mmol) was added. The reaction was stirred at room temperature for 1 hr. After the reaction was completed, the reaction solution was concentrated under reduced pressure, and the crude product was purified by running through a reversed-phase column (acetonitrile: water = 72%) to obtain t-butyl N-[(5S)-5-[(1-{[(2R)-3-(benzoxy)-1-{1-methansulfonyl-11,2-dihydrospiro[indol-3,4-piperidin] -1 -yl]-1-oxopropan-2-yl]carbamoyl}-1-methylethyl)carbamoyl]-5-{[(t-butoxy)carbonyl]amino}pentyl]carbamate **3a** (280 mg, white solid). Yield: 86%.

MS m/z (ESI): 857.4[M+1]⁺.

### Step II

### Preparation of (2S)-2,6-diamino-N-(1-{ [(2R)-3-(benzoxy)-1-{ 1-methansulfonyl-1,2-dihydrospiro[indol-3,4-piperidin]-1-yl]-1-oxopropan-2-yl]carbamoyl}-1-methylethyl)caproamide

T-butyl N-[(5S)-5-[(1-{[(2R)-3-(benzoxy)-1-{1-methansulfonyl-1,2-dihydrospiro[indol-3,4-piperidin]-1-yl]-1-oxopropan-2-yl]carbamoyl}-1-methylethyl)carbamoyl]-5- f [(t-butoxy)carbonyl]amino}pentyl]carbamate **3a** (280 mg, 0.33 mmol) was dissolved in dichloromethane (10 mL), and trifluoroacetic acid (3 mL) was added. The reaction was stirred at room temperature for 1 hr. After the reaction was completed, the reaction solution was concentrated under reduced pressure, and the crude product was purified by running through a reversed-phase column (acetonitrile : water =40%) to obtain (2S)-2,6-diamino-N-(1-{[(2R)-3-(benzoxy)-1-{1-methansulfonyl-1,2-dihydrospiro[indol-3,4-piperidin]-1-yl]-1-oxopropan-2-yl]carbamoyl}-1-methylethyl)caproamide **3**(210 mg, white solid). Yield: 97%.

MS m/z (ESI+): 657.3[M+1]⁺.

HPLC: 100% (214 nm), 100% (254 nm).

¹H NMR (400 MHz, *d*₆-DMSO) δ 8.61-8.48 (m, 1H), 8.12-8.09 (m, 2H), 7.84-7.78 (m, 3H), 7.61 (d, *J* = 8.0 Hz, 0.5H), 7.38-7.22 (m, 6H), 7.07-6.97 (m, 1H), 6.88 (d, *J* = 8.0 Hz, 0.5H), 4.99-4.96 (m, 1H), 4.51 (s, 1H), 4.47 (d, *J=* 9.2 Hz, 1H), 4.43-4.39 (m, 1H), 3.95-3.85 (m, 3H), 3.76 (s, 1H), 3.65 (dd, *J* = 16.4, 8.2 Hz, 1H), 3.59-3.48 (m, 1H), 3.34 (s, 3H), 3.21-3.11 (m, 1H), 3.05 (d, *J* = 6.4 Hz, 3H), 2.89-2.69 (m, 3H), 1.80-1.52 (m, 8H), 1.45 (d, *J* = 12.8 Hz, 3H), 1.39-1.36 (m, 4H).

### Example 4

### (S)-2-amino-N-(1-(((R)-3-(benzoxy)-1-(1-(methansulfonyl)spiro[indolin-3,4'-piperidin]-1'-yl)-1-oxopropan-2-yl)amino)-2-methyl-1-oxopropan-2-yl)-3-(1H-indol-2-yl)propionamide

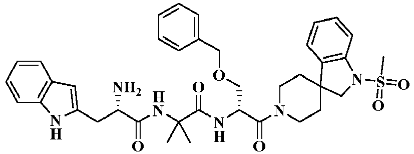

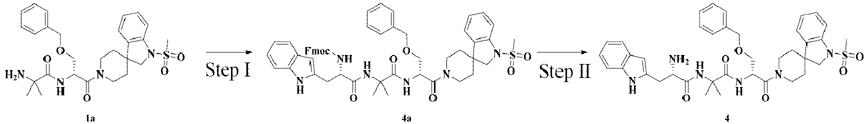

### Step I

### Preparation of 9H-fluoren-9-yl-methyl N-[(1S)-1-[(1-{[(2R)-3-(benzoxy)-1-{1-methansulfonyl-1,2-dihydrospiro[indol-3,4'-piperidin]-1'-yl]-1-oxopropan-2-yl]carbamoyl}-1-methylethyl)carbamoyl]-2-(1H-indol-2-yl)ethyl]carbamate

The compound Ibutamoren **1a** (200 mg, 0.38 mmol) was dissolved in DMF (3 mL), and then N,N-diiso-propylethyl amine (145 mg, 0.12 mmol), HATU (300 mg, 0.78 mmol) and ((9H-fluoro-9-yl)methoxy)carbonyl)-L-tryptophan (243 mg, 0.57 mmol) were added. The reaction solution was stirred at 25°C for 2 hrs. The reaction solution was quenched with water (20 mL), extracted with ethyl acetate (3 × 20 mL), washed with saturated brine (3 × 20 mL), and concentrated. The resulting residue was purified by preparative chromatography (acetonitrile/water) to obtain Compound **4a** (250 mg, white solid). Yield: 71%.

MS m/z (ESI): 937.0[M+1]⁺.

### Step II

### Preparation of (S)-2-amino-N-(1-(((R)-3-(benzoxy)-1-(1-(methansulfonyl)spiro[indolin-3,4'-piperidin]-1'-yl)-1-oxopropan-2-yl)amino)-2-methyl-1-oxopropan-2-yl)-3-(1H-indol-2-yl)propionamide

Compound **4a** (200 mg) was dissolved in acetonitrile (10 mL), and then piperidine (178 mg, 2.1 mmol) was added. The reaction solution was stirred at 25°C for 1 hr. The reaction solution was concentrated, and the resulting residue was purified by preparative chromatography (acetonitrile/water) to obtain Compound 4 (52.4 mg, white solid). Yield: 33%.

MS m/z (ESI): 715.0[M+1]⁺.

¹H NMR (400 MHz, DMSO) δ 10.84 (s, 1H), 8.14 (d, J = 12.4 Hz, 1H), 7.81 (dd, J = 17.7, 8.9 Hz, 1H), 7.57 (t, J = 9.3 Hz, 1H), 7.40 - 7.27 (m, 7H), 7.22 (dd, J = 16.0, 8.2 Hz, 2H), 7.16 (s, 1H), 7.05 (dd, J = 16.6, 10.7 Hz, 1H), 6.96 (dd, J = 20.2, 8.8 Hz, 2H), 5.11-4.89 (m, 1H), 4.54 - 4.46 (m, 2H), 4.37 (dd, J = 14.0, 7.9 Hz, 1H), 4.04 - 3.93 (m, 1H), 3.93 - 3.84 (m, 2H), 3.69 (dd, J = 10.3, 2.8 Hz, 1H), 3.60 - 3.47 (m, 1H), 3.41 (dd, J = 15.9, 6.2 Hz, 2H), 3.22 - 3.06 (m, 2H), 3.05 (d, J = 4.8 Hz, 3H), 2.83 - 2.70 (m, 2H), 1.67-1.50 (m, 4H), 1.38 (s, 6H).

### Example 5

### (4R)-7,7-dimethyl-4-(1-(methylsulfonyl)spiro[indol-3,4'-piperidin]-1'-carbonyl)-6,9-dioxo-1-phenyl-2,10-dioxa-5,8-diazadodecan-11-yl isobutyrate

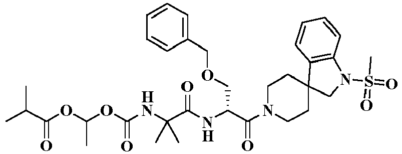

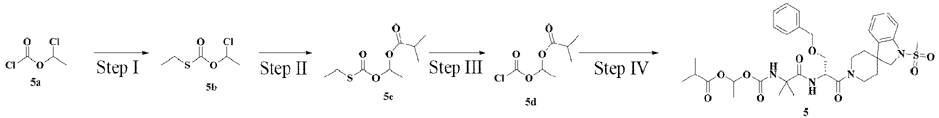

### Step I

### Preparation of O-(1-chloroethyl) S-ethyl thiocarbonate

Compound 1-chloroethyl chloroformate 5a (3 g, 0.02 mol) was dissolved in dichloromethane (10 mL), and then tetrabutylammonium bromide (TBAB) (0.34 g) was added. Sodium ethanethiolate (1.76 g; 0.02 mol) was dissolved in water (10 mL) and then added dropwise into the reaction solution. The reaction solution was stirred at 25°C for 16 hrs. The reaction solution was layered. The organic phase was washed with water (20 mL), dried over anhydrous sodium sulfate, and concentrated to obtain Compound 5b (2.0 g, yield: 56 %) as a yellow oil.

¹H NMR (400 MHz, CDCl₃) δ 6.60 (q, J = 5.8 Hz, 1H), 2.97-2.86 (m, 2H), 1.81 (d, J = 5.8 Hz, 3H), 1.34 (t, J = 7.4 Hz, 3H).

### Step II

### Preparation of 1-{[(ethylthio)carbonyl]oxy}ethyl isobutyrate

Compound **5b** (700 mg; 4.15 mmol) was dissolved in isobutyric acid (2.2 g, 25 mmol), and then N,N-diiso-propylethyl amine (1.6 g, 12.5 mmol) was added. The reaction solution was stirred at 55°C for 48 hrs. The reaction solution was quenched with water (20 mL), extracted with ethyl acetate (20 mL), washed with saturated sodium bicarbonate (3 × 30 mL) and then with saturated brine (2 × 20 mL), and concentrated to obtain a residue 5c (850 mg, pale yellow oil).

¹HNMR(400MHz,CDCl₃): δ 6.94 (q, J=5.4Hz, 1H), 2.92-2.83 (m, 2H), 2.59(dt, J=4.3, 2.4Hz, 1H), 1.50(d, J=5.5Hz, 3H),1.32(td, J=7.3, 3.6Hz, 3H), 1.20(d, J=7.0Hz, 6H).

### Step III

### Preparation of 1-((chlorocarbonyl)oxyethyl isobutyrate

Sulfonyl chloride (147 mg,1.09 mmol) was slowly added dropwise to Compound ethyl 1-{[(ethylthio)carbonyl]oxy}ethyl2-methylpropionate 5c(200 mg, 0.91mmol) at 0-5°C, and the reaction solution was stirred at 25°C for 45 min. The reaction solution was concentrated to obtain a residue 5d, which could be directly used in the next step.

### Step IV

### Preparation of 1- f [(1- { [(2R)-3-(benzoxy)-1- { 1-methansulfonyl-1,2-dihydrospiro[indol-3,4'-piperidin]-1'-yl}-1-oxopropan-2-yl]carbamoyl}-1-methylethyl)carbamoyl]oxy}ethyl 2-methylpropionate

Compound **5d** (60 mg, 0.11 mmol) and Ibutamoren **1a** was dissolved in dichloromethane (3 mL), and then sodium hydroxide (22 mg,0.22 mmol) was dissolved in water (5 mL) and slowly added dropwise to the reaction solution. The reaction solution was stirred at 25°C for 2 hrs. The organic layer was concentrated, and the obtained residue was purified by preparative chromatography (acetonitrile/water) to obtain Compound 5 (58 mg, white solid). Yield: 72%. MS m/z (ESI): 687.0[M+1]⁺.

¹HNMR(400MHz, MeOD) δ 7.79-7.59(m, 1H), 7.42-7.26(m, 6H), 7.25-7.16(m, 1H), 6.99-6.90(m, 1H), 6.81-6.66(m, 1H), 5.18-5.11(m, 1H), 4.59-4.49(m, 2H), 4.18-3.97(m, 1H), 3.99-3.84(m, 2H), 3.81-3.63(m, 2H), 3.26-3.16(m, 2H), 2.96(d,J=6.4Hz, 3H), 2.87-2.8 (m, 1H), 2.52-2.50(m, 1H), 2.06-1.55(m, 4H), 1.51-1.31(m, 9H), 1.11-1.02(m, 6H).

### Example 6

### (4R,11R)-7,7-dimethyl-4-(1-(methylsulfonyl)spiro[indol-3,4'-piperidin]-1'-carbonyl)-6,9-dioxo-1-phenyl-2,10-dioxa-5,8-diazadecan-11-yl isobutyrate

### (4R,11S)-7,7-dimethyl-4-(1-(methylsulfonyl)spiro[indol-3,4'-piperidin]-1'-carbonyl)-6,9-dioxo-1-phenyl-2,10-dioxa-5,8-diazadecan-11-yl isobutyrate

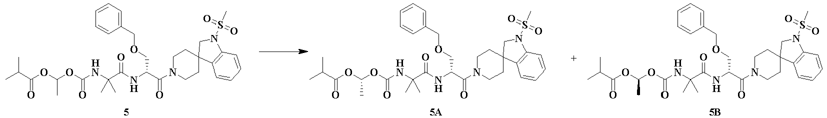

The prepared Compound 5 was chirally resolved to obtain Compound 5A and Compound 5B. Resolution condition: chromatographic column: Daicel CHIRALPAK IC_3, 3.0* 150 mm, 3 um, mobile phase: A/B: CO₂/MeOH=60/40, flow rate: 1.5 mL/min, column temperature: 37°C.

### Compound 5A:

t_{R}=1.582 min

MS m/z (ESI): 687.0[M+1]⁺.

¹H NMR (300 MHz, dmso) δ 7.80 - 7.49 (m, 2H), 7.42 - 7.15 (m, 8H), 7.08 - 6.85 (m, 2H), 6.67 - 6.56 (m, 1H), 4.97 (d, *J=* 7.2 Hz, 1H), 4.44 (dd, *J=* 30.4, 12.1 Hz, 3H), 3.90 (d, *J=* 6.8 Hz, 3H), 3.72 - 3.45 (m, 2H), 3.15 (s, 1H), 3.04 (d, *J=* 2.2 Hz, 3H), 2.80 (s, 1H), 1.66 (s, 4H), 1.45 - 1.28 (m, 9H), 1.06 (d, *J=* 6.9 Hz, 6H).

### Compound 5B:

t_{R}=1.815 min

MS m/z (ESI): 687.0[M+1]⁺.

¹H NMR (300 MHz, dmso) δ 7.77 - 7.50 (m, 2H), 7.42 - 7.15 (m, 8H), 7.09 - 6.85 (m, 2H), 6.67 - 6.57 (m, 1H), 4.97 (d, *J* = 7.2 Hz, 1H), 4.44 (dd, *J* = 28.5, 13.9 Hz, 3H), 3.97 (dd, *J* = 38.3, 7.0 Hz, 3H), 3.74 - 3.43 (m, 2H), 3.15 (s, 1H), 3.04 (d, *J=* 2.3 Hz, 3H), 2.80 (s, 1H), 1.66 (s, 3H), 1.45 - 1.30 (m, 9H), 1.09 - 1.02 (m, 6H).

### Example 7

### (R)-4-amino-N-(1-((3-(benzoxy)-1-(1-(methansulfonyl)spiro[indolin-3,4'-piperidin]-1'-yl)-1-oxopropan-2-yl)amino)-2-methyl-1-oxopropan-2-yl)butyl amine

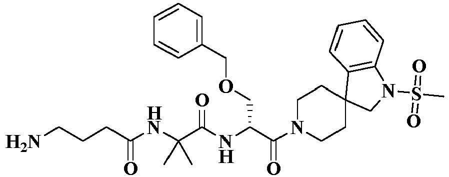

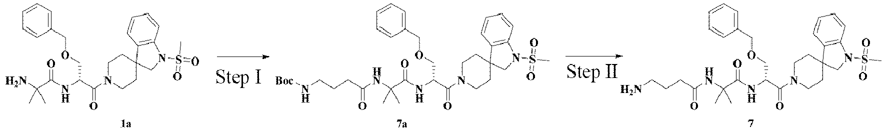

### Step I

### Preparation of t-butyl (R)-(4-((1-((3-(benzoxy)-1-(1-(methansulfonyl)spiro[indol-3,4'-piperidin]-1'-yl)-1-oxopropan-2-yl)amino)-2-methyl-1-oxopropan-2-yl)amino)-4-oxobutyl)carbamate

Ibutamoren 1a (150 mg, 0.28 mmol) was dissolved in dry DMF (5 mL), and then 4-((t-butoxycarbonyl)amino)butyric acid (69 mg, 0.34 mmol), HATU (320 mg, 0.84 mmol) and DIPEA (181 mg, 1.40 mmol) were added. The reaction solution was stirred at room temperature for 3 hrs. The reaction solution was purified by preparative chromatography (acetonitrile/water) to obtain t-butyl (R)-(4-((1-((3-(benzoxy)-1-(1-(methansulfonyl)spiro[indol-3,4'-piperidin]-1'-yl)-1-oxopropan-2-yl)amino)-2-methyl-1-oxopropan-2-yl)amino)-4-oxobutyl)carbamate 7a (162 mg, white solid). Yield: 80%.

MS m/z (ESI): 714.3.[M+1]⁺.

### Step II

### Preparation of (R) -4-amino-N-(1-((3-(benzoxy)-1-(1-(methansulfonyl)spiro[indolin-3,4'-piperidin]-1'-yl)-1-oxopropan-2-yl)amino)-2-methyl-1-oxopropan-2-yl)butyl amine

T-butyl (R)-(4-((1-((3-(benzoxy)-1-(1-(methansulfonyl)spiro[indol-3,4'-piperidin]-1'-yl)-1-oxopropan-2-yl)amino)-2-methyl-1-oxopropan-2-yl)amino)-4-oxobutyl)carbamate 7a (160 mg, 0.22 mmol) was dissolved in dichloromethane (5 mL), and then trifluoroacetic acid (0.5 mL) was added. The reaction solution was stirred at room temperature for 1 hr. The reaction solution was concentrated, and the obtained residue was purified by preparative chromatography (30% acetonitrile/water) to obtain (R) -4-amino-N-(1-((3-(benzoxy)-1-(1-(methansulfonyl)spiro[indolin-3,4'-piperidin]-1'-yl)-1-oxopropan-2-yl)amino)-2-methyl-1-oxopropan-2-yl)butyl amine 7 (28.1 mg, white solid). Yield: 21%.

MS m/z (ESI): 615.3[M+1]⁺.

¹H NMR (400 MHz, CDCl₃) *δ* 8.01 (s, 2H), 7.63-7.45 (m, 1H), 7.40-7.31 (m, 3H), 7.28 (s, 1H), 7.23 (d, *J* = 6.1 Hz, 2H), 7.11-6.92 (m, 1H), 6.70 (d, *J* = 7.5 Hz, 1H), 5.13 (br s, 1H), 4.59-4.42 (m, 2H), 4.04 (s, 1H), 3.87-3.74 (m, 2H), 3.73-3.55 (m, 2H), 3.20-3.08 (m, 1H), 3.06-2.82 (m, 4H), 2.79-2.76 (m, 1H), 2.47 (s, 2H), 2.01-1.98 (m, 2H), 1.92 (s, 1H), 1.89-1.83 (m, 3H), 1.70-1.63 (m, 2H), 1.50 (s, 6H).

HPLC: 99.71% (214 nm), 98.47% (254 nm).

### Example 8

### (R)-N-(1-((3-(benzoxy)-1-(1-(methansulfonyl)spiro[indolin-3,4'-piperidin]-1'-yl)-1-oxopropan-2-yl)amino)-2-methyl-1-oxopropan-2-yl)oleamide

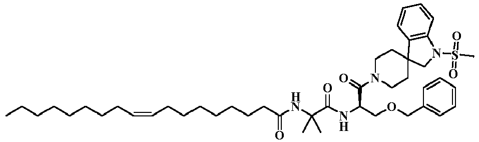

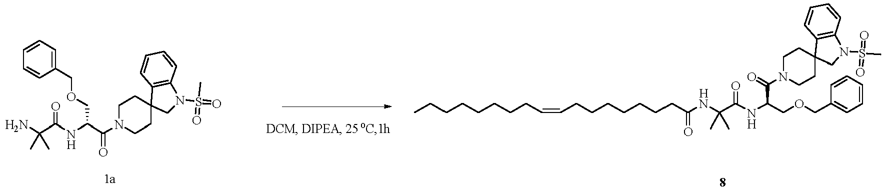

### Step I

### Preparation of (R) -N-(1-((3-(benzoxy)-1-(1-(methansulfonyl)spiro[indolin-3,4'-piperidin]-1'-yl)-1-oxopropan-2-yl)amino)-2-methyl-1-oxopropan-2-yl)oleamide

Compound Ibutamoren 1a (200 mg, 0.38 mmol) was dissolved in DCM (5 mL), and then N,N-diiso-propylethyl amine (147 mg, 1.14 mmol), HATU (288 mg, 0.76 mmol) and oleic acid (161 mg, 0.57 mmol) was added. The reaction solution was stirred at 25°C for 1 hr. The reaction solution was concentrated, and the resulting residue was purified by HPLC (acetonitrile/water) to obtain Compound **8** (150 mg, white solid). Yield: 47%.

MS m/z (ESI): 793.5 [M+1]⁺.

¹H NMR (400 MHz, CDCl3) δ 7.40 - 7.30 (m, 4H), 7.27 -2.25(m, 2H), 7.24 - 7.17 (m, 1H), 7.11 - 6.91 (m, 2H), 6.60 (d, J = 7.6 Hz, 1H), 5.99 (s, 1H), 5.43 - 5.26 (m, 2H), 5.21 - 5.04 (m, 1H), 4.70 - 4.41 (m, 3H), 4.19 - 3.91 (m, 1H), 3.90 - 3.65 (m, 3H), 3.59 -3.55(m, 1H), 3.18 - 2.98 (m, 1H), 2.90 (d, J = 4.6 Hz, 3H), 2.84 - 2.68 (m, 1H), 2.24 - 2.13 (m, 2H), 2.00-1.98 (m, 3H), 1.87 - 1.82 (m, 1H), 1.74 - 1.66 (m, 2H), 1.58-1.55 (m, 10H), 1.26 -1.20(m, 20H), 0.88 (t, J = 6.8 Hz, 3H).

### Example 9

### (Phosphoryloxy)methyl (R)-(1-((3-(benzoxy)-1-(1-(methansulfonyl)spiro[indol-3,4'-piperidin]-1'-yl)-1-oxopropan-2-yl)amino)-2-methyl-1-oxopropan-2-yl)carbamate

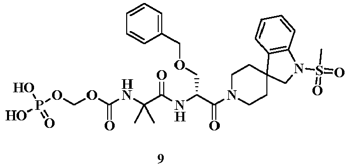

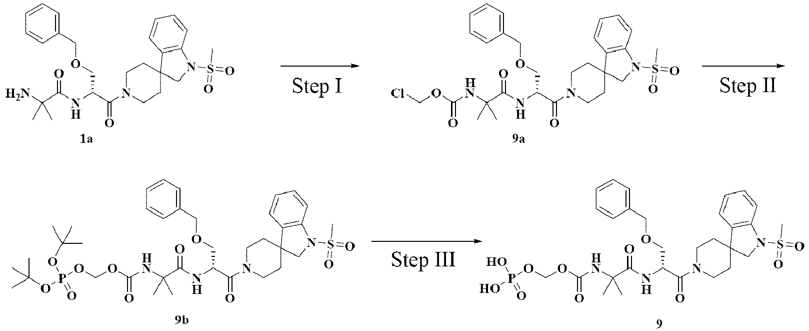

### Step I

### Preparation of chloromethyl (R)-(1-((3-(benzoxy)-1-(1-(methylsulfonyl)spiro[indolin-3,4'-piperidin]-1'-yl)-1-oxopropan-2-yl)carbamate

Ibutamoren **1a** (264 mg, 0.50 mmol) and chloromethyl choroformate (71 mg, 0.55 mmol) were dissolved in dichloromethane (10 mL), and then water (10 mL) and sodium hydroxide (40 mg, 1.0 mmol) were added. The reaction solution was stirred at room temperature for 2 hrs. The reaction solution was allowed to stand and be layered. The organic phase was dried and concentrated to obtain chloromethyl (R)-(1-((3-(benzoxy)-1-(1-(methylsulfonyl)spiro[indolin-3,4'-piperidin]-1'-yl)-1-oxopropan-2-yl)carbamate 9a (310 mg, colorless oil). Yield: 100%.

MS m/z (ESI): 621.2[M+1]⁺.

### Step II

### Preparation of ((di-t-butoxyphosphoryl)oxy)methyl (R)-(1-((3-(benzoxy)-1-(1-(methylsulfonyl)spiro[indol-3,4'-piperidin]-1'-yl)-1-oxopropan-2-yl)amino)-2-methyl-1-oxopropan-2-yl)carbamate

Chloromethyl (R)-(1-((3-(benzoxy)-1-(1-(methylsulfonyl)spiro[indolin-3,4'-piperidin]-1'-yl)-1-oxopropan-2-yl)carbamate 9a (310 mg, 0.50 mmol) was dissolved in dry tetrahydrofuran (10 mL), and then di-t-butyl tetra-n-butylammonium phosphate (678 mg, 1.50 mmol) and sodium iodide (225 mg, 1.50 mmol) were added. The reaction solution was stirred at room temperature for 24 hrs. The reaction solution was concentrated, and the resulting residue was purified by chromatography on silica gel (petroleum ether/ethyl acetate = 1/1) to obtain ((di-t-butoxyphosphoryl)oxy)methyl (R)-(1-((3-(benzoxy)-1-(1-(methylsulfonyl)spiro[indol-3,4'-piperidin]-1'-yl)-1-oxopropan-2-yl)amino)-2-methyl-1-oxopropan-2-yl)carbamate **9b** (200 mg, yellow oil). Yield: 50%.

MS m/z (ESI): 817.2[M+23]⁺.

### Step III

### Preparation of (phosphoryloxy)methyl (R)-(1-((3-(benzoxy)-1-(1-(methansulfonyl)spiro[indolin-3,4'-piperidin]-1'-yl)-1-oxopropan-2-yl)carbamate

((Di-t-butoxyphosphoryl)oxy)methyl (R)-(1-((3-(benzoxy)-1-(1-(methylsulfonyl)spiro[indol-3,4'-piperidin]-1'-yl)-1-oxopropan-2-yl)amino)-2-methyl-1-oxopropan-2-yl)carbamate**9b** (119 mg, 0.15 mmol) was dissolved in dichloromethane (10 mL), and then trifluoroacetic acid (1 mL) was added. The reaction solution was stirred at room temperature for 3 hrs. The reaction solution was concentrated, and the residue was purified by running through a reversed-phase column (35% acetonitrile/water) to obtain (phosphoryloxy)methyl (R)-(1-((3-(benzoxy)-1-(1-(methansulfonyl)spiro[indolin-3,4'-piperidin]-1'-yl)-1-oxopropan-2-yl)carbamate **9** (35.6 mg, white solid). Yield: 34%.

MS m/z (ESI): 683.2[M+1]⁺.

¹H NMR (400 MHz, DMSO) *δ* 7.83-7.62 (m, 2H), 7.44-7.10 (m, 7H), 6.98-6.85 (m, 2H), 5.4-5.32 (m, 2H), 4.97 (s, 1H), 4.56-4.25 (m, 3H), 3.88 (s, 3H), 3.59-3.52 (m, 2H), 3.23- 3.10 (m, 1H), 3.03 (s, 3H), 2.85-2.70 (m, 1H), 1.62 (br s, 4H), 1.36 (br s, 6H).

### Example 10

### ((Diethoxyphosphoryl)oxy)methyl (R)-(1-((3-(benzoxy)-1-(1-(methylsulfonyl)spiro[indol-3,4'-piperidin]-1'-yl)-1-oxopropan-2-yl)amino)-2-methyl-1-oxopropan-2-yl)carbamate

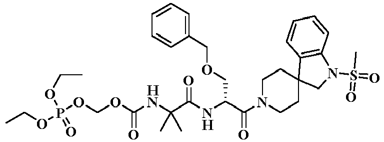

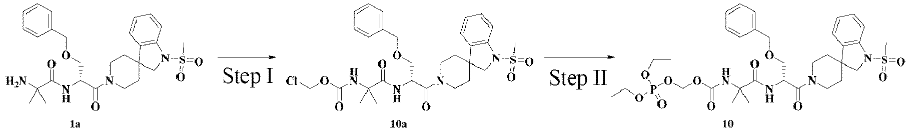

### Step I

### Preparation of chloromethyl (R)-(1-((3-(benzoxy)-1-(1-(methylsulfonyl)spiro[indolin-3,4'-piperidin]-1'-yl)-1-oxopropan-2-yl)carbamate

Ibutamoren **1a** (264 mg, 0.50 mmol) and chloromethyl choroformate (71 mg, 0.55 mmol) were dissolved in dichloromethane (10 mL), and then water (10 mL) and sodium hydroxide (40 mg, 1.0 mmol) were added. The reaction solution was stirred at room temperature for 2 hrs. The reaction solution was allowed to stand and be layered. The organic phase was dried and concentrated to obtain chloromethyl (R)-(1-((3-(benzoxy)-1-(1-(methylsulfonyl)spiro[indolin-3,4'-piperidin]-1'-yl)-1-oxopropan-2-yl)carbamate **10a** (310 mg, colorless oil). Yield: 100%.

MS m/z (ESI): 621.3[M+1]⁺.

### Step II

### Preparation of ((diethoxyphosphoryl)oxy)methyl (R)-(1-((3-(benzoxy)-1-(1-(methylsulfonyl)spiro[indol-3,4'-piperidin]-1'-yl)-1-oxopropan-2-yl)amino)-2-methyl-1-oxopropan-2-yl)carbamate

Chloromethyl (R)-(1-((3-(benzoxy)-1-(1-(methylsulfonyl)spiro[indolin-3,4'-piperidin]-1'-yl)-1-oxopropan-2-yl)carbamate **10a** (310 mg, 0.50 mmol) was dissolved in dry tetrahydrofuran (10 mL), and then the compound diethyl tetra-n-butylammonium phosphate (593 mg, 1.50 mmol) and sodium iodide (225 mg, 1.50 mmol) were added. The reaction solution was stirred at room temperature for 24 hrs. The reaction solution was concentrated, and the obtained residue was purified by preparative chromatography (55% acetonitrile/water) to obtain ((diethoxyphosphoryl)oxy)methyl (R)-(1-((3-(benzoxy)-1-(1-(methylsulfonyl)spiro[indol-3,4'-piperidin]-1'-yl)-1-oxopropan-2-yl)amino)-2-methyl-1-oxopropan-2-yl)carbamate **10** (86 mg, white solid). Yield: 12%.

MS m/z (ESI): 739.2[M+1]⁺.

¹H NMR (400 MHz, CDCl₃): *δ* 7.43-7.27 (m, 5.5H), 7.27-7.17 (m, 2H), 7.11-6.96 (m, 2H), 6.63 (d, *J* = 7.4 Hz, 0.5H), 5.82 (s, 1H), 5.59 (d, *J* = 13.5 Hz, 2H), 5.20-5.13 (m, 1H), 4.69-4.42 (m, 3H), 4.22-4.07 (m, 4H), 3.89-3.65 (m, 3H), 3.63-3.58 (m, 1H), 3.17-3.01 (m, 1H), 2.91 (d, *J=* 4.7 Hz, 3H), 2.85-2.68 (m, 1H), 1.84-1.67 (m, 4H), 1.59-1.57 (m, 6H), 1.34 (t, *J=* 7.0 Hz, 6H).

³¹P NMR (162 MHz, CDCl₃): *δ* -2.59 (s, 1H).

### Example 11

### (4R)-7,7-dimethyl-4-(1-(methylsulfonyl)spiro[indolin-3,4'-piperidin]-1'-carbonyl)-6,9-dioxo-1-phenyl-2,10-dioxa-5,8-diazadodecan-11-yl propionate

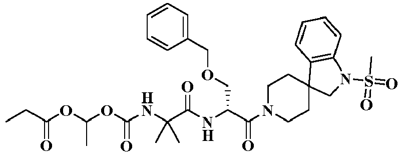

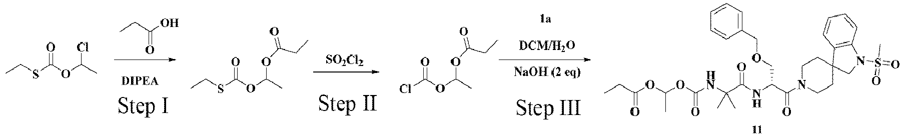

### Step I

### Preparation of 1-((ethylthio)carbonyl)oxyethyl propionate

O-(1-chloroethyl) S-ethylthiocarbonate (2 g, 0.0119 mol) was dissolved in propionic acid (4.4 g, 0.0595 mol), and then *N*,*N*-diiso-propylethyl amine(4.6 g, 0.0357 mol) was added. The resulting solution was stirred at 55°C for 48 hrs, and then the reaction was terminated, and washed with water (30 mL) and ethyl acetate (30 mL). The organic phase was washed with a saturated NaHCO₃ solution (3 × 30 mL) and then with brine (1 × 30 mL). The solvent was removed by distillation, to obtain the desired product 1-((ethylthio)carbonyl)oxyethyl propionate (850 mg, yellow oil). Yield: 83%.

¹H NMR (400 MHz, CDCl₃) *δ* 6.94 (q, *J* = 5.4 Hz, 1H), 2.92 - 2.83 (m, 2H), 2.59 (dt, *J* = 4.3, 2.4 Hz, 1H), 1.50 (d, *J* = 5.5 Hz, 3H), 1.32 (td, *J* = 7.3, 3.6 Hz, 3H), 1.20 (d, *J* = 7.0 Hz, 6H).

### Step II

### (Chlorocarbonyl)oxyethyl propionate

Sulfonyl chloride (147 mg, 1.089 mmol) was added to 1-((ethylthio)carbonyl)oxyethyl propionate (200 mg, 0.907 mmol), and stirred at 0-5°C for 5 min. After all the reagents were added, the reaction solution was stirred at room temperature for 45 min. Then, EtSCl was removed by distillation at room temperature. The product was directly used in the next step without further purification.

### Step III

### (4R)-7,7-dimethyl-4-(1-(methylsulfonyl)spiro[indolin-3,4'-piperidin]-1'-carbonyl)-6,9-dioxo-1-phenyl-2,10-dioxa-5,8-diazadodecan-11-yl propionate

To a round bottom flask, Ibutamoren **1a** (50 mg, 0.09 mmol) and (chlorocarbonyl)oxyethyl propionate (51 mg, 0.28 mmol) were added, and then dichloromethane (15 mL) were added. Then, sodium hydroxide (19 mg, 0.19 mmol) was added to water (5 mL), mixed and stirred at room temperature for 2 hrs. The organic layer was collected, concentrated, and then purified by preparative chromatography (chromatographic column: Kromasil-C18 100 × 21.2 mm, mobile phase: acetonitrile-water over gradient: 30 - 40) to obtain (4R)-7,7-dimethyl-4-(1-(methylsulfonyl)spiro[indolin-3,4'-piperidin]-1 '-carbonyl)-6,9-dioxo-1 -phenyl-2,10-dioxa-5,8-diazadodecan-11-yl propionate 11 (23 mg, yellow solid). Yield: 35%.

MS m/z (ESI): 673.2[M+1]⁺.

¹H NMR (400 MHz, DMSO) δ 7.65 (dd, J = 34.3, 11.5 Hz, 2H), 7.52 (d, J = 7.5 Hz, 1H), 7.31 - 7.26 (m, 5H), 7.01 (t, J = 7.3 Hz, 1H), 6.94 (d, J = 7.3 Hz, 1H), 6.85 (t, J = 7.0 Hz, 1H), 6.61 (d, J = 5.3 Hz, 1H), 4.94 (d, J = 6.1 Hz, 1H), 4.48 - 4.43 (m, 2H), 3.93 - 3.60 (m, 6H), 3.01 (d, J = 3.9 Hz, 5H), 1.63 (s, 4H), 1.34 (dd, J = 10.5, 5.2 Hz, 4H), 1.29 (s, 6H), 0.98 - 0.93 (m,4H).

### Example 12

### (4R)-7,7-dimethyl-4-(1-(methylsulfonyl)spiro[indol-3,4'-piperidin]-1'-carbonyl)-6,9-dioxo-1-phenyl-2,10-dioxa-5,8-diazadodecan-11-yl 2-methylbutyrate

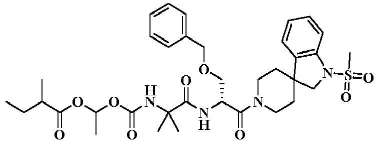

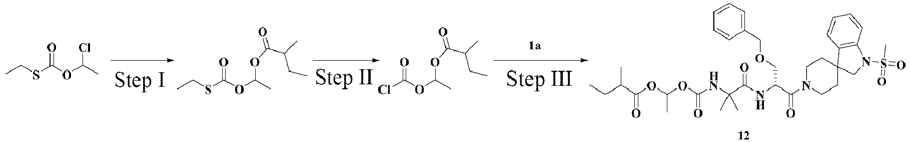

### Step I

### Preparation of 1-((ethylthio)carbonyloxy)ethyl 2-methylbutyrate

(1-Chloroethoxy)(ethylsulfanilamido)methanone (2.00 g, 11.86 mmol) was dissolved in 2-methylbutyric acid (6.5 mL, 59.30 mmol), and then N,N-diiso-propylethyl amine (4.60 g, 35.58 mmol) was added. The reaction solution was reacted at 55°C for 48 hrs. Then, the reaction solution was cooled to room temperature and extracted with water and ethyl acetate. The organic phase was washed with a saturated sodium carbonate solution and then with saturated brine, dried, and concentrated to obtain 1-((ethylthio)carbonyloxy)ethyl 2-methylbutyrate (1.4 g, yellow oil). Yield: 50%.

¹H NMR (400 MHz, CDCl₃) δ 6.95 (q, J = 5.4 Hz, 1H), 2.90-2.84 (m, 2H), 2.41-2.35 (m, 1H), 1.74-1.62 (m, 1H), 1.51 (d, J = 5.5 Hz, 3H), 1.48-1.41 (m, 1H), 1.31 (t, J = 7.4 Hz, 3H), 1.15 (dd, J = 7.0, 3.5 Hz, 3H), 0.91 (td, J = 7.4, 1.8 Hz, 3H).

### Step II

### Preparation of 1-((chlorocarbonyl)oxy)ethyl 2-methylbutyrate

At 0°C, sulfonyl chloride (138 mg, 1.02 mmol) was added dropwise to 1-((ethylthio)carbonyloxy)ethyl 2-methylbutyrate (200 mg, 0.85 mmol). The reaction solution was stirred at room temperate for 1 hr. The reaction solution was concentrated under reduced pressure to obtain crude 1-((chlorocarbonyl)oxy)ethyl 2-methylbutyrate (178 mg, yellow oil), which was directly used in the next step.

### Step III

### Preparation of (4R)-7,7-dimethyl-4-(1-(methylsulfonyl)spiro[indol-3,4'-piperidin]-1'-carbonyl)-6,9-dioxo-1-phenyl-2,10-dioxa-5,8-diazadodecan-11-yl 2-methylbutyrate

Ibutamoren 1a (50 mg, 0.095 mmol) and 1-((chlorocarbonyl)oxy)ethyl 2-methylbutyrate (28 mg, 0.28 mmol) were dissolved in dichloromethane (5 mL), and then water (5 mL) and sodium hydroxide (19 mg, 0.19 mmol) were added. The reaction solution was stirred at room temperature for 2 hrs. The reaction solution was allowed to stand and be layered. The organic phase was dried and concentrated, and the obtained residue was purified by running through a reversed-phase column (55% acetonitrile/water) to obtain (4R)-7,7-dimethyl-4-(1-(methylsulfonyl)spiro[indol-3,4'-piperidin]-1'-carbonyl)-6,9-dioxo-1-phenyl-2,10-dioxa-5,8-diazadodecan-11-yl 2-methylbutyrate **12** (9.1 mg, white solid). Yield: 13%.

MS m/z (ESI): 701.2[M+1]⁺.

¹H NMR (400 MHz, CDCl₃) *δ* 7.39-7.28 (m, 5.5H), 7.23-7.17 (m 1H), 7.07-6.96 (m, 2H), 6.79-6.78 (m, 1H), 6.64-6.61 (m, 0.5H), 5.53-5.42 (m, 1H), 5.23-5.09 (m, 1H), 4.69-4.39 (m, 3H), 4.10-3.97 (m, 1H), 3.91-3.51 (m, 4H), 3.16-3.02 (m, 1H), 2.90 (d, *J=* 4.4 Hz, 3H), 2.87-2.73 (m, 1H), 2.40-2.32 (m, 1H), 1.96-1.77 (m, 2H), 1.74-1.64 (m, 4H), 1.56-1.54 (m, 6H), 1.45 (t, *J=* 5.7 Hz, 3H), 1.13 (dd, *J=* 6.9, 1.8 Hz, 3H), 0.90 (t, *J=* 7.4 Hz, 3H).

### Example 13

### (4R)-7,7-dimethyl-4-(1-(methylsulfonyl)spiro[indolin-3,4'-piperidin] -1'-carbonyl)-6,9-dioxo-1-phenyl-2,10-dioxa-5,8-diazadodecan-11-yl cyclohexylcarboxylate

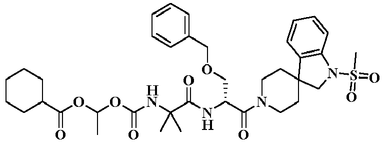

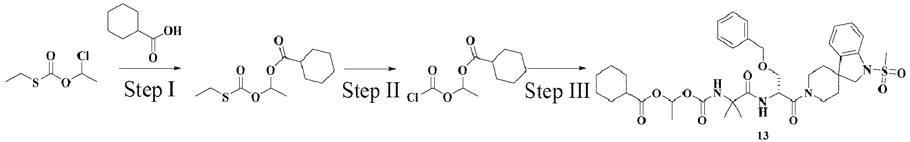

### Step I

### Preparation of 1-(((ethylthio)carbonyl)oxy)ethyl cyclohexylcarboxylate

O-(1-chloroethyl) S-ethylthiocarbonate (2 g, 0.0119 mol) was dissolved in cyclohexylcarboxylic acid (7.6 g, 0.0595 mol), and then *N*,*N*-diiso-propylethyl amine (4.6 g,0.0357 mol) was added. The resulting solution was stirred at 55°C for 48 hrs. Then the reaction was terminated, and washed with water (30 mL) and ethyl acetate (30 mL). The organic phase was washed with a saturated sodium bicarbonate solution (3 × 30 mL), and then with saturated brine (2 × 30 mL). The solvent was removed by distillation, to obtain the desired product 1-((((ethylthio)carbonyl)oxy)ethyl cyclohexylcarboxylate (2g, yellow oil). Yield: 54%.

¹H NMR (400 MHz, CDCl₃) *δ* 6.93 (q, *J* = 5.4Hz,1 H), 2.91 - 2.82 (m, 2 H), 2.36 - 2.29 (m, 1 H), 1.90 (dd, *J=* 14.1, 3.1Hz,2 H), 1.77 - 1.72 (m, 2 H), 1.71 - 1.52 (m, 2 H), 1.51 - 1.48 (m, 3 H), 1.47 - 1.41 (m, 2 H), 1.33 - 1.30 (m, 3 H), 1.25 (dd, *J=* 9.7, 2.7 Hz,2 H).

### Step II

### Preparation of 1-((chlorocarbonyl)oxy)ethyl cyclohexylcarboxylate

Sulfonyl chloride (125 mg, 0.922 mmol) was added to 1-((((ethylthio)carbonyl)oxy)ethyl cyclohexylcarboxylate (200 mg, 0.77 mmol), and the mixture was stirred at 0-5°C for 5 min. After all the reagents were added, the reaction solution was stirred at room temperature for 45 min. Then, EtSCl was removed by distillation at room temperature. The product was directly used in the next step without further purification.

### Step III

### Preparation of (4R)-7,7-dimethyl-4-(1-(methylsulfonyl)spiro[indolin-3,4'-piperidin] -1'-carbonyl)-6,9-dioxo-1-phenyl-2,10-dioxa-5,8-diazadodecan-11-yl cyclohexylcarboxylate

To a round bottom flask, Ibutamoren **1a**(50 mg, 0.09 mmol) and [1-(cyclohexyloxy)ethyl] chloroformate (66.6 mg, 0.28 mmol) was dissolved in dichloromethane (15 mL). Then, NaOH (19.2 mg, 0.19 mmol) was added to water (5 mL), added dropwise to the reaction solution, and mixed with stirring for 2 hrs at room temperature. The organic layer was purified by preparative chromatography (chromatographic column: Kromasil-C18 100 × 21.2 mm 5; mobile phase: acetonitrile-water gradient over: 30 - 40) to obtain (4R)-7,7-dimethyl-4-(1-(methylsulfonyl)spiro[indolin-3,4'-piperidin] -1'-carbonyl)-6,9-dioxo-1-phenyl-2,10-dioxa-5,8-diazadodecan-11-yl cyclohexylcarboxylate **13** (23 mg, yellow solid). Yield: 33%.

MS m/z (ESI): 727.2[M+1]⁺.

¹H NMR (400 MHz, DMSO) *δ* 7.74 (s, 1H), 7.65 (d, *J* = 12.0 Hz, 1H), 7.57 (d, *J* = 8.7 Hz, 1H), 7.35 - 7.22 (m, 5H), 7.04 (s, 1H), 6.98 (d, *J* = 7.0 Hz, 1H), 6.87 (s, 1H), 4.96 (d, *J* = 7.1 Hz, 1H), 4.46 (dd, *J* = 22.7, 19.3 Hz, 2H), 3.90 (d, *J* = 11.8 Hz, 2H), 3.55 (d, *J* = 24.5 Hz, 2H), 3.04 (d, *J* = 4.2 Hz, 3H), 2.54 - 2.52 (m, 4H), 2.31 - 2.24 (m, 1H), 1.79 (t, *J* = 41.2 Hz, 4H), 1.61 (d, *J* = 39.3 Hz, 6H), 1.37 (dd, *J* = 28.3, 15.6 Hz, 9H), 1.26 - 1.02 (m, 4H).

### Example 14

### (4R)-7,7-dimethyl-4-(1-(methylsulfonyl)spiro[indol-3,4'-piperidin]-1'-carbonyl)-6,9-dioxo-1-phenyl-2,10-dioxa-5,8-diazadodecan-1 1-yl acetate

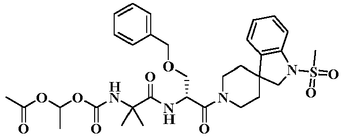

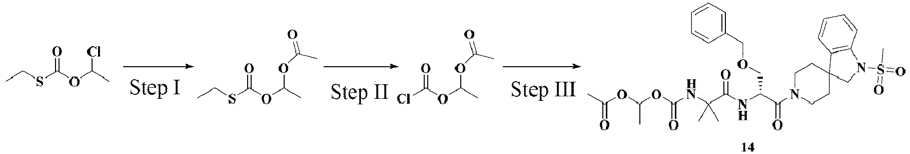

### Step I

### Preparation of 1-((ethylthio)carbonyl)oxyethyl acetate

(1-chloroethoxy)(ethylsulfanilamido)methanone (2.00 g, 11.86 mmol) was dissolved in acetic acid (3.6 mL, 59.30 mmol), and then N,N-diiso-propylethyl amine (4.6 g, 35.58 mmol) was added. The reaction was stirred at 55°C for 48 hrs. Then, the reaction solution was cooled to room temperature and extracted with water and ethyl acetate. The organic phase was washed with a saturated sodium carbonate solution and then with saturated brine, dried, and concentrated to obtain 1-((ethylthio)carbonyl)oxyethyl acetate (1.2 g, yellow oil). Yield: 53%.

¹H NMR (400 MHz, CDCl₃) δ 6.94 (q, J = 5.5 Hz, 1H), 2.95-2.81 (m, 2H), 2.09 (s, 3H), 1.51 (d, J = 5.5 Hz, 3H), 1.32 (t, J = 7.4 Hz, 3H).

### Step II

### Preparation of (chlorocarbonyl)oxyethyl acetate

Sulfonyl chloride (253 mg, 1.87 mmol) was added dropwise at 0°C to 1-((ethylthio)carbonyl)oxyethyl acetate (300 mg, 1.56 mmol), and the reaction solution was stirred at room temperature for 1 hr. The reaction solution was concentrated under reduced pressure to obtain crude (chlorocarbonyl)oxyethyl acetate (178 mg, yellow oil). Yield: 100%. The crude product was directly used in the next step.

### Step III

### Preparation of (4R)-7,7-dimethyl-4-(1-(methylsulfonyl)spiro[indol-3,4'-piperidin]-1'-carbonyl)-6,9-dioxo-1-phenyl-2,10-dioxa-5,8-diazadodecan-11-yl acetate

Ibutamoren **1a** (50 mg, 0.095 mmol) and (chlorocarbonyl)oxyethyl acetate (47 mg, 0.28 mmol) were dissolved in dichloromethane (5 mL), and then water (5 mL) and sodium hydroxide (19 mg, 0.19 mmol) were added. The reaction solution was stirred at room temperature for 2 hrs. The reaction solution was allowed to stand and be layered. The organic phase was dried and concentrated, and the obtained residue was purified by running through a reversed-phase column (55% acetonitrile/water) to obtain (4R)-7,7-dimethyl-4-(1-(methylsulfonyl)spiro[indol-3,4'-piperidin]-1'-carbonyl)-6,9-dioxo-1-phenyl-2,10-dioxa-5,8-diazadodecan-11-yl acetate **14** (16.27 mg, white solid). Yield: 26%.

MS m/z (ESI): 659.2[M+1]⁺.

¹H NMR (400 MHz, CDCl₃) *δ* 7.46-7.27 (m, 5H), 7.25-7.17 (m, 1H), 7.14-6.90 (m, 2H), 6.81-6.73 (m, 1H), 6.73-6.46 (m, 1H), 5.43 (s, 1H), 5.21-5.13 (m, 1H), 4.71-4.39 (m, 3H), 4.15-3.94 (m, 1H), 3.92-3.52 (m, 4H), 3.17-3.02 (m, 1H), 2.89 (d, *J* = 4.4 Hz, 3H), 2.83-2.62 (m, 1H), 2.06 (d, *J=* 4.3 Hz, 3H), 1.93-1.84 (m, 1H), 1.71-1.69 (m, 3H), 1.54 (d, *J=* 5.9 Hz, 6H), 1.46 (t, *J* = 5.5 Hz, 3H).

### Example 15

### (R)-5,5-dimethyl-8-(1-(methylsulfonyl)spiro[indolin-3,4'-piperidin] -1'-carbonyl)-3,6-dioxo-11-phenyl-2,10-dioxa-4,7-diazadodecyl isobutyrate

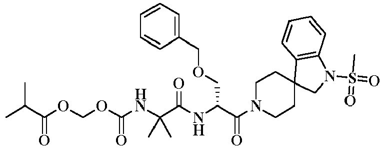

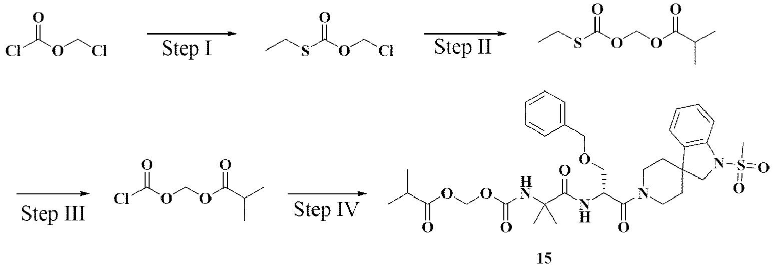

### Step I

### Preparation of O-(chloromethyl) S-ethylthiocarbonate

Sodium ethanethiolate (3.07 g, 36.5 mmol) was dissolved in dichloromethane (20 ml), then tetrabutylammonium bromide (0.59 g, 1.8 mmol) was added, and chloromethyl choroformate (4.71 g, 36.5 mmol) was slowly added dropwise. The reaction was stirred at room temperature for 24 hrs. After the reaction was completed, the reaction solution was extracted with dichloromethane (2 × 30 mL), and washed with saturated brine. The organic phase was collected, and then dried over anhydrous sodium sulfate, and the solvent was removed by rotary evaporation to obtain O-(chloromethyl) S-ethylthiocarbonate (3 g, yellow oil liquid). Yield: 50%.

¹H NMR (400 MHz, DMSO) δ 5.96 (s, 1H), 2.96 - 2.87 (m, 1H), 1.31 - 1.20 (m, 2H).

### Step II

### Preparation of (((ethylthio)carbonyl)oxy)methyl isobutyrate

Isobutyric acid (8.95 g, 97 mmol) was added to an eggplant-shaped flask, and then O-(chloromethyl) S-ethylthiocarbonate (3 g, 19.4 mmol) was added, followed by N,N-diisopropylethyl amine(6.9 g, 53 mmol). The reaction was stirred at 55°C for 48 hrs. After the reaction was completed, a sodium bicarbonate solution (15 mL) was added to the reaction solution. Then, the system was extracted with dichloromethane (2 × 30 mL) and washed with saturated brine. The organic phase was collected, and then dried over anhydrous sodium sulfate, and the solvent was removed by rotary evaporation to obtain ((((ethylthio)carbonyl)oxy)methyl isobutyrate (1.85 g, orange oil liquid). Yield: 44%.

¹H NMR (400 MHz, DMSO) δ 5.78 (s, 2H), 4.03 (q, *J=* 7.2 Hz, 3H), 2.88 (q, *J=* 7.2 Hz, 1H), 2.66 - 2.55 (m, 1H), 1.20 - 1.15 (m, 4H), 1.10 (d, *J=* 7.2 Hz, 6H).

### Step III

### ((Chlorocarbonyl)oxy)methyl isobutyrate

((((Ethylthio)carbonyl)oxy)methyl isobutyrate (500 mg, 2.42 mmol) was added to an eggplant-shaped flask, and then sulfonyl chloride was added (1 mL). The reaction was stirred at room temperature for 1 hr. After the reaction was completed, the solvent was removed by rotary evaporation to obtain crude ((chlorocarbonyl)oxy)methyl isobutyrate (437 mg, brown solid). Yield: 99%. The crude product was used directly in the next reaction without purification. Step IV

### Preparation of (R)-5,5-dimethyl-8-(1-(methylsulfonyl)spiro[indolin-3,4'-piperidin] -1'-carbonyl)-3,6-dioxo-11-phenyl-2,10-dioxa-4,7-diazadodecyl isobutyrate

((Chlorocarbonyl)oxy)methyl isobutyrate (437 mg, 2.42 mmol) was dissolved in dichloromethane (10 mL), and Compound Ibutamoren 1a (200 mg, 0.38 mmol) was dissolved in dichloromethane (5 mL). The two solutions were combined, and water (5 mL) and a 2N sodium hydroxide solution (0.2 mL) were added. The reaction was stirred at room temperature for 16 hrs. After the reaction was completed, the reaction solution was extracted with dichloromethane (2 × 30 mL), and washed with saturated brine. The organic phase was collected, and then dried over anhydrous sodium sulfate, and the solvent was removed by rotary evaporation to obtain a crude product. The crude product was purified and separated by column chromatography (acetonitrile: water (0.1% formic acid)) to obtain (R)-5,5-dimethyl-8-(1-(methylsulfonyl)spiro[indolin-3,4'-piperidin]-1'-carbonyl)-3,6-dioxo-11-phenyl-2,10-dioxa-4,7-diazadodecyl isobutyrate 15 (34 mg, white solid). Yield: 2%.

MS m/z (ESI) 673.3[M+1]⁺.

1H NMR (400 MHz, DMSO) δ 7.76 (dd, *J* = 16.7, 11.1 Hz, 2H), 7.59 (d, *J* = 8.0 Hz, 1H), 7.34 (s, 8H), 7.01 (dt, *J* = 19.7, 7.2 Hz, 1H), 6.89 (d, *J* = 7.4 Hz, 0.5H), 5.60 (s, 2H), 4.97 (dd, *J* = 13.2, 6.6 Hz, 1H), 4.51 (s, 1H), 4.47 (d, *J* = 4.1 Hz, 1H), 4.44 - 4.33 (m, 1H), 3.94 - 3.85 (m, 1H), 3.70 - 3.62 (m, 1H), 3.61 - 3.54 (m,1H), 3.50 (dd, *J=* 9.4, 5.4 Hz, 1H), 3.17 (dd, *J* = 25.5, 12.3 Hz, 1H), 3.04 (d, *J* = 3.9 Hz, 3H), 2.80 (t, *J* = 11.5 Hz, 1H), 1.61 (dd, *J* = 34.8, 11.5 Hz, 1H), 1.34 (s,2H), 1.07 (t, *J* = 5.7 Hz, 2H).

### Example 16

### (R)-2-(2-aminoacetamido)-N-(3-(benzoxy)-1-(1-(methansulfonyl)spiro[indolin-3,4'-piperidin]-1'-yl)-1-oxopropan-2-yl)-2-methyl propionamide

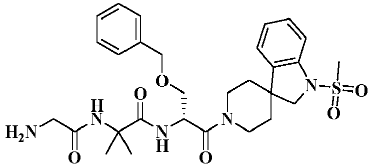

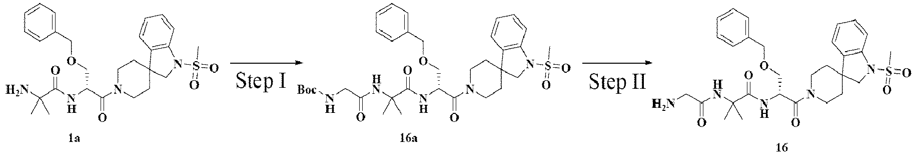

### Step I

### Preparation of t-butyl (R)-(2-((1-((3-(benzoxy)-1-(1-(methansulfonyl)spiro[indolin-3,4'-piperidin]-1'-yl)-1-oxopropan-2-yl)amino)-2-methyl-1-oxopropan-2-yl)carbamate

Ibutamoren **1a** (212 mg, 0.40 mmol) was dissolved in dry DMF (5 mL), and then BOC-glycine (105 mg, 0.60 mmol), HATU (457 mg, 1.20 mmol) and DIPEA (259 mg, 2.00 mmol) were added. The reaction solution was stirred at room temperature for 3 hrs. The reaction solution was purified by preparative chromatography (55% acetonitrile/water) to obtain t-butyl (R)-(2-((1-((3-(benzoxy)-1-(1-(methansulfonyl)spiro[indolin-3,4'-piperidin]-1'-yl)-1-oxopropan-2-yl)amino)-2-methyl-1-oxopropan-2-yl)carbamate **16a** (220 mg, white solid). Yield: 80%.

MS m/z (ESI): 686.3[M+1]⁺.

### Step II

### Preparation of (R)-2-(2-aminoacetamido)-N-(3-(benzoxy)-1-(1-(methansulfonyl)spiro[indolin-3,4'-piperidin]-1'-yl)-1-oxopropan-2-yl)-2-methylpropionamide

T-butyl (R)-(2-((1-((3-(benzoxy)-1-(1-(methansulfonyl)spiro[indolin-3,4'-piperidin]-1'-yl)-1-oxopropan-2-yl)amino)-2-methyl-1-oxopropan-2-yl)carbamate **16a** (150 mg, 0.22 mmol) was dissolved in dichloromethane (5 mL), and then trifluoroacetic acid (1 mL) was added. The reaction solution was stirred at room temperature for 3 hrs. The reaction solution was concentrated, and the obtained residue was purified by preparative chromatography (30% acetonitrile/water) to obtain (R) -2-(2-aminoacetamido)-N-(3-(benzoxy)-1-(1-(methansulfonyl)spiro[indolin-3,4'-piperidin]-1'-yl)-1-oxopropan-2-yl)-2-methyl propionamidel6 (35 mg, white solid). Yield: 27%.

MS m/z (ESI): 586.3[M+1]⁺.

¹H NMR (400 MHz, DMSO) *δ* 8.37 (d, *J* = 3.6 Hz, 1H), 7.97-7.94 (m, 3H), 7.79 (d, *J* = 8.2 Hz, 0.5H), 7.42-7.15 (m, 6H), 7.12-6.95 (m, 1H), 6.90 (d, *J* = 7.5 Hz, 0.5H), 5.01-4.94 (m, 1H), 4.62-4.45 (m, 2H), 4.42-4.39 (m, 1H), 4.06-3.81 (m, 3H), 3.79-3.68 (m, 1H), 3.63-3.43 (m, 3H), 3.21-3.13 (m, 1H), 3.05 (d, *J* = 5.9 Hz, 3H), 2.84-2.76 (m, 1H), 1.84-1.54 (m, 4H), 1.49-1.23 (m, 6H).

### Example 17

### (5-methyl-2-oxo-1,3-dioxacyclopenten-4-yl)methyl (R)-(1-((3-(benzoxy)-1-(1-(methylsulfonyl)spiro[indol-3,4'-piperidin]-1'-yl)-1-oxopropan-2-yl)amino)-2-methyl-1-oxopropan-2-yl)carbamate

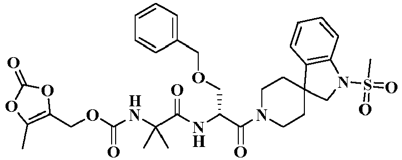

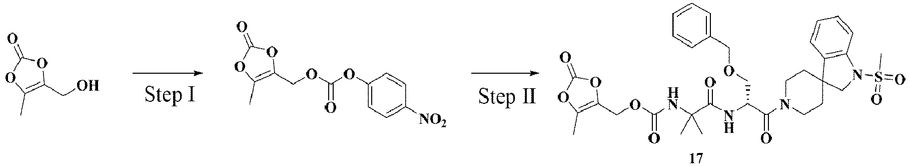

### Step I

### Preparation of (5-methyl-2-oxo-1,3-dioxacyclopenten-4-yl)methyl (4-nitrophenyl) carbonate

4-(hydroxymethyl)-5-methyl-1,3-dioxacyclopenten-2-one (1 g, 7.7 mmol) and pyridine (0.67 g, 8.47 mmol) were dissolved in dichloromethane (10 mL). 4-nitrophenyl chloroformate (1.55 g, 7.7 mmol) was dissolved in dichloromethane (20 mL), and added dropwise to the above solution in an ice bath. The reaction was stirred at room temperature for 16 hrs. After the reaction was completed, the reaction was washed sequentially with 1% sodium hydroxide aqueous solution, 1 N hydrochloric acid, water and saturated brine. The organic phase was collected, and then dried over anhydrous sodium sulfate, and the solvent was removed by rotary evaporation to obtain (5-methyl-2-oxo-1,3-dioxacyclopenten-4-yl)methyl (4-nitrophenyl) carbonate (2.4 g, yellow solid). Yield: 79%.

¹H NMR (400 MHz, DMSO) δ 8.36 - 8.30 (m, 2H), 7.64 - 7.54 (m, 2H), 5.19 (s, 2H), 2.25 - 2.11 (s, 3H).

### Step II

### Preparation of (5-methyl-2-oxo-1,3-dioxacyclopenten-4-yl)methyl (R)-(1-((3-(benzoxy)-1-(1-(methylsulfonyl)spiro[indol-3,4'-piperidin]-1'-yl)-1-oxopropan-2-yl)amino)-2-methyl-1-oxopropan-2-yl)carbamate

Ibutamoren **1a** (107 mg, 0.2 mmol) was dissolved in dichloromethane (5 mL), and then (5-methyl-2-oxo-1,3-dioxacyclopenten-4-yl)methyl (4-nitrophenyl) carbonate (200 mg, 0.81 mmol) and pyridine (64 mg, 0.81 mmol) were added. The reaction was stirred at room temperature for 16 hrs. After the reaction was completed, the reaction solution was extracted with dichloromethane (2 × 30 mL), and washed with saturated brine. The organic phase was collected, and then dried over anhydrous sodium sulfate, and the solvent was removed by rotary evaporation to obtain a crude product. The crude product was separated and purified by running through a reversed-phase column (acetonitrile/water (containing 0.1%formic acid)) to obtain (5-methyl-2-oxo-1,3-dioxacyclopenten-4-yl)methyl (R)-(1-((3-(benzoxy)-1-(1-(methylsulfonyl)spiro[indol-3,4'-piperidin]-1'-yl)-1-oxopropan-2-yl)amino)-2-methyl-1-oxopropan-2-yl)carbamate **17** (48 mg, white solid). Yield: 8%.

MS m/z (ESI) 685.3[M+1]⁺.

¹H NMR (400 MHz, DMSO) δ 7.76 (d, *J* = 8.4 Hz, 0.5H), 7.59 (d, *J =* 16.4 Hz, 2H), 7.42 - 7.17 (m, 6H), 7.03 (dd, *J* = 22.9, 7.1 Hz, 1H), 6.89 (d, *J* = 7.6 Hz, 0.5H), 4.96 (m, 1H), 4.82 (d, J = 10.9 Hz, 1H), 4.51 (s, 1H), 4.47 (d, *J* = 7.1 Hz, 1H), 4.41 (d, *J* = 14.4 Hz, 1H), 3.90 (d, *J =* 10.5 Hz, 1H), 3.63 (d, *J* = 7.2 Hz, 1H), 3.55(m, 1H), 3.49 (m, 1H), 3.14 (m, 1H), 3.04 (d, *J* = 4.3 Hz, 3H), 2.79 (m, 2H), 2.10 (d, *J* = 7.7 Hz, 3H), 2.04 - 1.93 (m, 1H), 1.64 (d, *J* = 12.9 Hz, 1H), 1.34 (s, 3H), 1.23 (s, 3H).

### Example 18

### (R)-3-((1-((3-(benzoxy)-1-(1-(methansulfonyl)spiro[indolin-3,4'-piperidin]-1'-yl)-1-oxopropan-2-yl)amino)-2-m ethyl-1 -oxopropan-2-yl)carbamoyl)-1 -methylpyridin-1 -ium quaternary ammonium salt

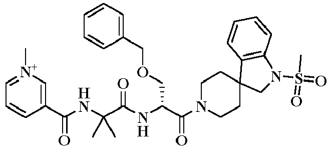

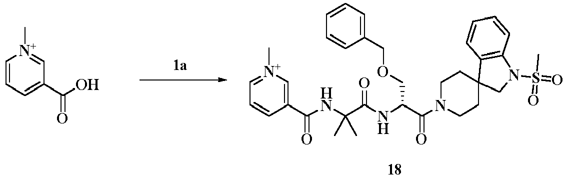

### Step I

### Preparation of (R)-3-((1-((3-(benzoxy)-1-(1-(methansulfonyl)spiro[indolin-3,4'-piperidin]-1'-yl)-1-oxopropan-2-yl)amino)-2-methyl-1-oxopropan-2-yl)carbamoyl)-1-methylpyridin-1-ium chloride

Trigonelline hydrochloride (985 mg, 5.67 mmol) was dissolved in N,N-dimethylformamide (10 mL), and then Ibutamoren **1a**(500 mg, 0.95 mmol), N,N-diisopropylethyl amine (611 mg, 4.73 mmol) and 1-propylphosphoric anhydride (903 mg, 2.84 mmol) were added. The reaction was stirred at room temperature for 48 hrs. After the reaction was completed, the reaction solution was directly purified and separated by running through a reversed-phase column (acetonitrile/water), to obtain (R) -3-((1-((3-(benzoxy)-1-(1-(methansulfonyl)spiro[indolin-3,4'-piperidin]-1'-yl)-1-oxopropan-2-yl)amino)-2-methyl-1-oxopropan-2-yl)carbamoyl)-1-methylpyridin-1-ium quaternary ammonium salt **18** (352 mg, white solid). Yield: 54%.

MS m/z (ESI): 648.3[M+1]⁺.

¹H NMR (400 MHz, DMSO) δ 9.61 (s, 2H), 9.09 (s, 1H), 8.92 (s, 1H), 8.39 (d, *J* = 8.1 Hz, 0.5H), 8.15 (d, *J* = 7.9 Hz, 1H), 7.36 - 7.19 (m, 7H), 7.15 (d, *J* = 7.3 Hz, 0.5H), 6.98 (dt, *J* = 12.9, 6.9 Hz, 1H), 4.93 (m, 1H), 4.48 (m, 1H), 4.44 (d, *J=* 12.9 Hz, 4H), 3.89 (m, 3H), 3.72 (d, *J* = 7.7 Hz, 2H), 3.53 (dd, *J* = 9.2, 5.5 Hz, 1H), 3.46 (dd, *J* = 9.1, 5.1 Hz, 1H), 3.17 (dd, *J* = 25.6, 12.3 Hz, 1H), 3.04 (s, 3H), 2.76 (t, *J=* 12.2 Hz, 1H), 1.86 (m, 2H), 1.64 (s, 2H), 1.49 (s, 6H).

### Example 19

### (R,Z)-N-(3-(benzoxy)-1-(1-(methylsulfonyl)spiro[indol-3,4'-piperidin]-1'-yl)-1-oxopropan-2-yl)-2-methyl-2-((4-oxopen-2-en-2-yl)amino)propionamide

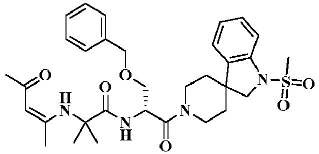

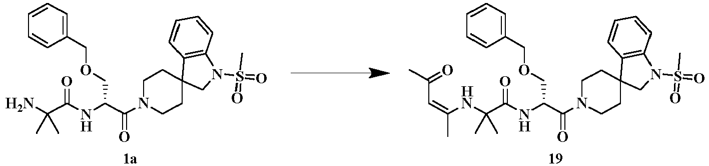

### Step I

### Preparation of (R,Z)-N-(3-(benzoxy)-1-(1-(methylsulfonyl)spiro[indol-3,4'-piperidin]-1'-yl)-1-oxopropan-2-yl)-2-methyl-2-((4-oxopen-2-en-2-yl)amino)propionamide

In a sealed tube, Ibutamoren **1a** (150 mg, 0.28 mmol) was dissolved in acetylacetone (2 mL). The sealed tube was positioned in a microwave reactor, heated to 130°C with microwave radiation at 150W, and stirred for 10 min. After the reaction was completed, the reaction solution was directly purified and separated by running through a reversed-phase column (acetonitrile/water (containing 0.1% NH₃)), to obtain (R, Z)-N-(3-(benzoxy)-1-(1-(methylsulfonyl)spiro[indol-3,4'-piperidin]-1'-yl)-1-oxopropan-2-yl)-2-methyl-2-((4-oxopen-2-en-2-yl)amino)propionamide 19(48 mg, white solid). Yield: 26%.

MS m/z (ESI): 611.3[M+1]⁺.

¹H NMR (400 MHz, DMSO) δ 8.15 (d, *J* = 8.2 Hz, 1H), 8.07 (d, *J* = 8.3 Hz, 1H), 7.43-7.15 (m, 7H), 7.07 (s, 1H), 7.00 (s, 1H), 6.66 (s, 2H), 5.34 - 5.29 (m, 1H), 5.00 (s, 2H), 4.51 (s, 1H), 4.48 (d, *J* = 6.3 Hz, 1H), 4.37 (d, *J=* 12.5 Hz, 2H), 3.90 (m, 3H), 3.19 (m, 2H), 3.05 (d, *J* = 3.3 Hz, 3H), 2.00 (dd, *J* = 14.2, 6.7 Hz, 2H), 1.90 (s, 2H), 1.80 (d, *J* = 7.7 Hz, 2H), 1.44 (s, 3H), 1.23 (s, 6H).

### Example 20

### (4R)-7,7-dimethyl-4-(1-(methylsulfonyl)spiro[indolin-3,4'-piperidin] -1'-carbonyl)-6,9-dioxo-1-phenyl-2,10-dioxa-5,8-diazadodecan-11-yl pivalate

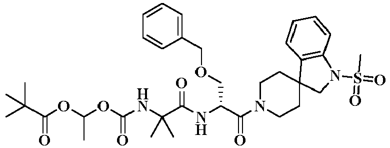

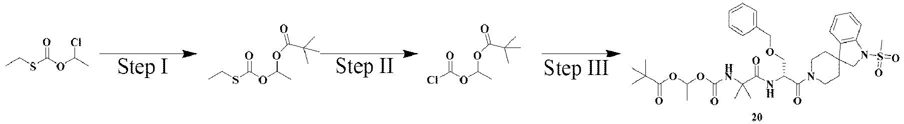

### Step I

### Preparation of 1-(((ethylthio) carbonyl)oxy)ethyl pivalate

O-(1-chloroethyl) S-ethylthiocarbonate (2 g, 11.90 mmol) was dissolved in 2,2-dimethylpropionic acid (7.29 g, 71.40 mmol), and then N,N-diiso-propylethyl amine (4.61 g, 35.70 mmol) was added. The reaction solution was reacted at 70 °C for 48 hrs. After the reaction was completed, water (100 mL) was added, and then the reaction solution was extracted with dichloromethane (3 × 50 mL). The organic phases were separated and combined, then washed with a saturated sodium bicarbonate solution (3 × 50 mL) and then with a saturated sodium chloride solution (100 mL), and dried over anhydrous sodium sulfate. The reaction solution was concentrated to obtain 1-(((ethylthio)carbonyl)oxy)ethyl pivalate (1.20 g, yellow oil). Yield: 39%.

¹H NMR (400 MHz, CDCl₃) *δ* 6.94-6.90 (m, 1H), 2.90-2.84 (m, 2H), 1.50 (d, *J* = 5.2 Hz, 3H), 1.31 (t, *J* = 7.2 Hz, 3H), 1.20 (s, 9H).

### Step II

### Preparation of 1-((chlorocarbonyl)oxy)ethyl pivalate

Sulfonyl chloride (766 mg, 5.67 mmol) was slowly added to 1-(((ethylthio) carbonyl) oxy)ethyl pivalate (665 mg, 2.84 mmol). The reaction solution was stirred at 0 °C for 5 min. Then the reaction solution was stirred at room temperature for 1 hr. The reaction solution was rotary dried to obtain crude 1-((chlorocarbonyl)oxy)ethyl pivalate, which was directly used in the next reaction without purification.

### Step III

### (4R)-7,7-dimethyl-4-(1-(methylsulfonyl) spiro[indolin-3,4'-piperidin] -1'-carbonyl)-6,9-dioxo-1-phenyl-2,10-dioxa-5,8-diazadodecan-11-yl pivalate

(R)-2-amino-N-(3-(benzoxy)-1-(1-(methylsulfonyl)spiro[indolin-3,4'-piperidin]-1'-yl)-1-oxopropan-2-yl)-2-methyl propionamide (200 mg, 0.72 mmol) and 1-((chlorocarbonyl)oxy)ethyl pivalate (600 mg, 1.12 mmol) were dissolved in dichloromethane (15 mL), and then a solution of sodium hydroxide (272 mg, 6.80 mmol) in water (10 mL) was added. The reaction solution was reacted at 25 °C for 1 hr. After the reaction was completed, the reaction solution was extracted with dichloromethane (2 × 30 mL). The organic phases were combined, dried over anhydrous sodium sulfate, and concentrated. The resulting residue was purified by preparative chromatography to obtain (4R)-7,7-dimethyl-4-(1-(methylsulfonyl) spiro[indolin-3,4'-piperidin]-1'-carbonyl)-6,9-dioxo-1-phenyl-2,10-dioxa-5,8-diazadodecan-11-yl pivalate (79.0 mg, white solid). Yield: 30%.

MS m/z (ESI): 400.1[M+1]⁺.

¹H NMR (400 MHz, CDCl₃) *δ* 7.40-7.32 (m, 5H), 7.29 (s, 1H), 7.23-7.16 (m, 1H), 7.10-7.02 (m, 1H), 7.00-6.95 (m, 1H), 6.76-6.72 (m, 1H), 6.63-6.61 (m, 1H), 5.46-5.38 (m, 1H), 5.21-5.10 (m, 1H), 4.66-4.57 (m, 1H), 4.55-4.45 (m, 2H), 4.11-3.97 (m, 1H), 3.89-3.68 (m, 3H), 3.62-3.56 (m, 1H), 3.16-3.01 (m, 1H), 2.91 (d, *J* = 3.6 Hz, 3H), 2.87-2.74 (m, 1H), 1.93-1.82 (m, 1H), 1.75-1.65 (m, 3H), 1.62 (br, s, 3H), 1.45 (t, *J* = 5.6 Hz, 3H), 1.19 (s, 9H).

### Example 21

### (R)-5,5-dimethyl-8-(1-(methylsulfonyl)spiro[indolin-3,4'-piperidin] -1'-carbonyl)-3,6-dioxo-11-phenyl-2,10-dioxa-4,7-diazadodecyl pivalate

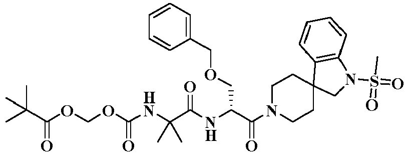

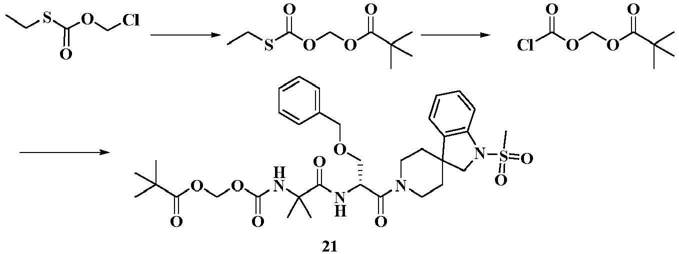

### Step I

### Preparation of (((ethylthio)carbonyl)oxy)methyl pivalate

Pivalic acid (1.65 g, 16.17 mmol) was dissolved in N,N-dimethylformamide (5 mL), then O-(chloromethyl) S-ethylthiocarbonate (500 mg, 3.23 mmol) was added, and then N,N-diisopropylethyl amine (1.25 g, 9.70 mmol) was added. The reaction was stirred at 55°C for 48 hrs. After the reaction was completed, a sodium bicarbonate solution (15 mL) was added to the reaction solution. Then, the system was extracted with dichloromethane (2 × 30 mL) and washed with saturated brine. The organic phase was collected, and then dried over anhydrous sodium sulfate, the solvent was removed by rotary evaporation to obtain (((ethylthio)carbonyl)oxy)methyl pivalate (600 mg, pale yellow oil liquid). Yield: 80%.

¹H NMR (400 MHz, CDCl₃) δ 5.81 (s, 2H), 2.89 (q, *J* = 7.4 Hz, 2H), 1.34 (d, *J* = 7.4 Hz, 3H), 1.22 (s, 9H).

### Step II

### Preparation of ((chlorocarbonyl)oxy)methyl pivalate

(((Ethylthio)carbonyl)oxy)methyl pivalate (400 mg, 1.82 mmol) was added to an eggplant-shaped flask, and then sulfonyl chloride (1 mL) was added. The reaction was stirred at room temperature for 1 hr. After the reaction was completed, the solvent was removed by rotary evaporation to obtain crude ((chlorocarbonyl)oxy)methyl pivalate (400 mg, brown solid), which was directly used in the next reaction without purification.

### Step III

### Preparation of (R)-5,5-dimethyl-8-(1-(methylsulfonyl)spiro[indolin-3,4'-piperidin] -1'-carbonyl)-3,6-dioxo-11-phenyl-2,10-dioxa-4,7-diazadodecyl isobutyrate

((Chlorocarbonyl)oxy)methyl pivalate (400 mg, 2.06 mmol) was dissolved in dichloromethane (10 mL), and Compound Ibutamoren (200 mg, 0.38 mmol) was dissolved in dichloromethane (5 mL). The two solutions were combined, and water (5 mL) and a 2N sodium hydroxide solution (0.2 mL) were added. The reaction was stirred at room temperature for 16 hrs. After the reaction was completed, the reaction solution was extracted with dichloromethane (2 × 30 mL), and washed with saturated brine. The organic phase was collected, and then dried over anhydrous sodium sulfate, and the solvent was removed by rotary evaporation to obtain a crude product. The crude product was purified and separated by preparative chromatography (acetonitrile: water (containing 0.1% TFA)) to obtain (R)-5,5-dimethyl-8-(1-(methylsulfonyl)spiro[indolin-3,4'-piperidin]-1'-carbonyl)-3,6-dioxo-11-phenyl-2,10-dioxa-4,7-diazadodecyl pivalate (50 mg, white solid). Yield: 18%.

MS m/z (ESI) 687.3[M+1]⁺.

¹H NMR (400 MHz, DMSO) δ 7.82 - 7.69 (m, 1H), 7.57 (d, *J* = 7.9 Hz, 0.5H), 7.38 - 7.19 (m, 8H), 7.06 - 6.96 (m, 1H), 6.88 (d, *J* = 7.5 Hz, 0.5H), 5.59 (s, 2H),4.97 (d, *J* = 6.7 Hz, 1H), 4.51 (s, 1H), 4.47 (d, *J* = 4.3 Hz, 1H), 4.44 - 4.34 (m, 2H), 3.70 - 3.62 (m, 1H), 3.61 - 3.53 (m, 1H), 3.50 (dd, *J* = 9.5, 5.2 Hz, 1H), 3.16(dd, *J* = 25.0, 12.2 Hz, 2H), 3.04 (d, *J* = 4.4 Hz, 3H), 1.66 (m, 4H), 1.34 (s, 6H), 1.13 (d, J = 4.3 Hz, 9H).

### Example 22

### (R)-5,5-dimethyl-8-(1-(methylsulfonyl)spiro[indolin-3,4'-piperidin] -1'-carbonyl)-3,6-dioxo-11-phenyl-2,10-dioxa-4,7-diazadodecyl acetate

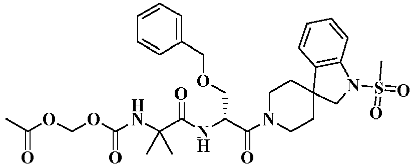

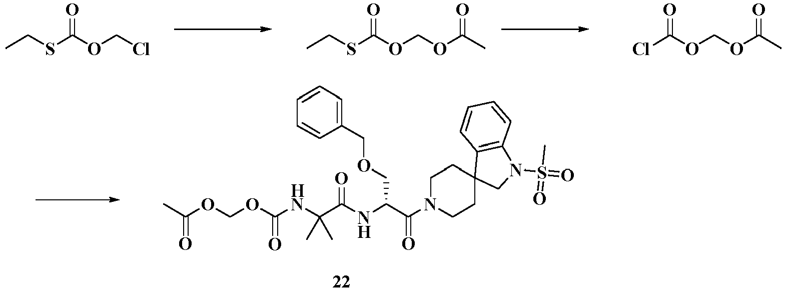

### Step I

### Preparation of (((ethylthio)carbonyl)oxy)methyl acetate

Acetic acid (1.95 g, 32.5 mmol) was added to an eggplant-shaped flask, and then O-(chloromethyl) S-ethylthiocarbonate (1 g, 6.5 mmol) was added, followed by N,N-diisopropylethyl amine(2.52 g, 19.5 mmol). The reaction was stirred at 55°C for 48 hrs. After the reaction was completed, a sodium bicarbonate solution (15 mL) was added to the reaction solution. Then, the system was extracted with ethyl acetate (2 × 30 mL) and washed with saturated brine. The organic phase was collected, and then dried over anhydrous sodium sulfate, and the solvent was removed by rotary evaporation to obtain (((ethylthio)carbonyl)oxy)methyl acetate (826 mg, pale yellow oil liquid). Yield: 65%.

¹H NMR (400 MHz, CDCl₃) δ 5.80 (s, 2H), 2.90 (q, *J* = 7.4 Hz, 2H), 2.13 (s, 3H), 1.36-1.32 (m, 3H).

### Step II

### Preparation of ((chlorocarbonyl)oxy)methyl acetate

(((Ethylthio)carbonyl)oxy)methyl acetate (200 mg, 1.23 mmol) was added to an eggplant-shaped flask, and then sulfonyl chloride (1 mL) was added. The reaction was stirred at room temperature for 1 hr. After the reaction was completed, the solvent was removed by rotary evaporation to obtain crude ((chlorocarbonyl)oxy)methyl acetate (172 mg, pale yellow oil liquid). The crude product was directly used in the next reaction without purification.

### Step III

### (R)-5,5-dimethyl-8-(1-(methylsulfonyl)spiro[indolin-3,4'-piperidin] -1'-carbonyl)-3,6-dioxo-11-phenyl-2,10-dioxa-4,7-diazadodecyl acetate

((Chlorocarbonyl)oxy)methyl acetate (173mg, 1.13 mmol) was dissolved in dichloromethane (10 mL), and Compound Ibutamoren (200 mg, 0.38 mmol) was dissolved in dichloromethane (5 mL). The two solutions were combined, and water (5 mL) and a 2N sodium hydroxide solution (0.2 mL) were added. The reaction was stirred at room temperature for 16 hrs. After the reaction was completed, the reaction solution was extracted with dichloromethane (2 × 30 mL), and washed with saturated brine. The organic phase was collected, and then dried over anhydrous sodium sulfate, and the solvent was removed by rotary evaporation to obtain a crude product. The crude product was separated and purified by preparative chromatography (acetonitrile: water (containing 0.1% FA)) to obtain (R)-5,5-dimethyl-8-(1-(methylsulfonyl)spiro[indolin-3,4'-piperidin]-1'-carbonyl)-3,6-dioxo-11-phenyl-2,10-dioxa-4,7-diazadodecyl acetate (45 mg, white solid). Yield: 18%.

MS m/z (ESI) 645.3[M+1]⁺.

¹H NMR (400 MHz, DMSO) δ 7.81 (d, *J* = 7.9 Hz, 1H), 7.73 (d, *J* = 13.8 Hz, 1H), 7.62 (d, *J* = 8.2 Hz, 0.5H), 7.34 (t, *J* = 3.5 Hz, 2H), 7.32 - 7.19 (m, 5H), 7.07 - 6.96 (m, 2H), 6.91 (d, *J* = 7.6 Hz, 0.5H), 5.57 (s, 2H), 5.00 - 4.94 (m, 1H), 4.52 (s, 1H), 4.47 (d, *J* = 4.4 Hz, 1H), 3.93 - 3.86 (m, 2H), 3.70 - 3.63 (m, 1H), 3.58 (d, *J* = 5.6 Hz, 1H), 3.54 - 3.45 (m, 1H), 3.17 (d, *J* = 12.1 Hz, 2H), 3.04 (d, *J* = 3.7 Hz, 3H), 2.79 (d, *J* = 10.7 Hz, 1H), 2.04 (d, *J* = 5.0 Hz, 3H), 1.74 - 1.59 (m, 4H), 1.34 (s, 6H).

### Example 23

### (R)-3-(3-(benzoxy)-1-(1-(methylsulfonyl)spiro[indolin-3,4'-piperidin] -1'-yl)-1-oxopropan-2-yl)-2,2,5,5-tetramethylimidazolin-4-one

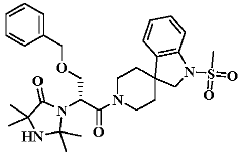

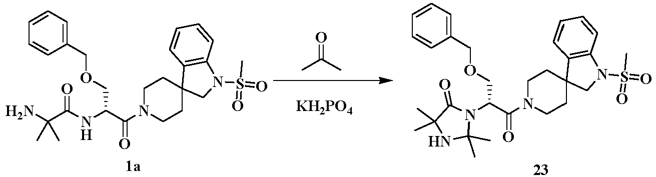

### Step I

Compound Ibutamoren 1a (1.056 g, 2 mmol) was dissolved in acetone (5 mL), and then potassium dihydrogen phosphate (54mg, 0.4mmol) was added, and stirred at 80°C for 5 hrs. Then, a new product was produced as detected by TLC. After cooling to room temperature, the reaction solution was extracted three times with ethyl acetate/water, and purified by column chromatography (petroleum ether: ethyl acetate=1:3) to obtain the target product **23** (1 g). Yield: 87%.

MS m/z (ESI): 570.28[M+1]⁺.

¹H NMR (300 MHz, DMSO-d6) δ 7.40-7.19 (m, 8H), 7.05 (t, *J* = 7.3 Hz, 1H), 5.15 (t, *J* = 7.1 Hz, 1H), 4.60-4.38 (m, 3H), 3.93-3.62 (m, 5H), 3.04 (s, 3H), 2,95-2.60 (m, 2H), 1.92-1.80 (m, 1H), 1.75-1.60 (m, 3H), 1.45-1.15(m, 12H).

### Example 24

### 3-[(2R)-3-(benzoxy)-1-{1-methansulfonyl-1,2-dihydrospiro[indol-3,4'-piperidin]-1'-yl}-1-oxopropan-2-yl]-5,5-dimethylimidazolin-2,4-dione

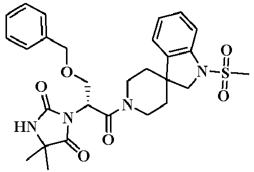

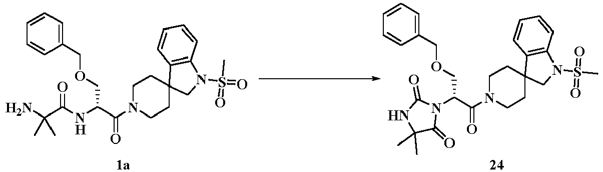

### Step I

### Preparation of 3-[(2R)-3-(benzoxy)-1-{1-methansulfonyl-1,2-dihydrospiro[indol-3,4'-piperidin]-1'-yl}-1-oxopropan-2-yl]-5,5-dimethylimidazolin-2,4-dione

Compound Ibutamoren**1a** (200 mg, 0.38 mmol) was dissolved in dichloromethane (5 mL), and then triethyl amine (115 mg, 1.12 mmol) and ethyl chloroformate (30 mg, 0.13 mmol) were slowly added to the reaction solution and stirred at 25 °C for 2 hrs. The reaction solution was concentrated, and the resulting residue was purified by preparative chromatography (acetonitrile/water) to obtain Compound **24**(70 mg, white solid). Yield: 33%.

MS m/z (ESI): 555.0[M+1]⁺.

¹H NMR (400 MHz, MeOD) δ 7.34-7.30 (m, 5H), 7.28 - 7.19 (m, 2H), 7.18 - 7.10 (m, 1H), 7.04 (t, J = 7.3 Hz, 1H), 5.21 (dd, J = 9.5, 5.3 Hz, 1H), 4.59-4.48 (m, 3H), 4.25 (t, J = 10.1 Hz, 1H), 4.07 - 3.88 (m, 3H), 3.81-3.79 (m, 1H), 3.29 - 3.19 (m, 1H), 2.96 (s, 3H), 2.87-2.82 (m, 1H), 1.85-1.75 (m, 4H), 1.35 (d, J = 10.0 Hz, 6H).

### Biological evaluation

### Test Example 1. Determination of human GHSR agonist activity of the compound of the present invention

This method was used to determine the agonism effect of the compound of the invention on the activity of human GHSR protein expressed in human GHSR/CHO stable cell line.

### I. Test materials and instruments

1. Culture medium
   F12 (Gibco, Cat# 11765-047);
   FBS (Corning, Cat# 35-076-CV);
   Geneticin (Invitrogen, Cat# 10131);
   Penicillin/Streptomycin (Invitrogen, Cat# 15140).
2. Reagents
   Fluo-4 Direct (Invitrogen, Cat# F10471);
   HBSS (Gibco, Cat# 14025076);
   HEPES (Gibco, Cat# 15630080);
   Bonine Serum Albumin (Sgima, Cat# B2064-100G).
3. Instruments
   384 well Poly-D-Lysine protein coating plate (Greiner, Cat# 781946);
   FLIPR (Molecular Devices);
   Vi-cell XR Cell Viability Analyzer (Beckman Coulter);
   Incubator (Thermo).

### II. Experimental steps

Preparation of compound gradient: Ghrelin and the compound of the present invention were 5-fold diluted to prepare 10 concentration gradients, and then transferred to a compound plate in an amount of 900 nL/well.

Preparation of buffer: HBSS (1X):HEPES (1M)=49:1, containing 0.5% BSA.

Cells of human GHSR/CHO stable cell line were inoculated into a 384-well plate, and incubated overnight. Then, the cell plate was removed, and the medium was discarded. 20 uL buffer as slowly added to each well, and then 20 uL 2X Fluo-4 Direct^{™} No-wash Loading Buffer was added to each well. The cell plate was incubated in an incubator at 37°C and 5% CO₂ for 50 min, and then removed and allowed to stand at room temperature for 10 min. 30 uL buffer was added to each well in the compound plate; and a buffer plate was additionally prepared and 30 uL buffer was added to each well. Test for agonism of compound: An FLIPR instrument and running software were used. 10 uL buffer was transferred to the cell plate and the value of fluorescence signal was read. 10 uL compound was transferred to the cell plate and the value of fluorescence signal was read. An FLIPR program was used to calculate the difference between the maximum value and the minimum value from the 91st signal point to the 230th signal point. The EC₅₀ value of the compound can be calculated by software from fluorescence values corresponding to various concentrations.

### III. Experimental results:

The human GHSR agonist activity of the compound of the present invention is determined through the above experiments, and the obtained EC₅₀ values are shown in Table 1.

**Table 1. EC₅₀ value of human GHSR agonist activity of the compound of the present invention**

| Compound No. | EC₅₀ (nM) |
|---|---|
| Ghrelin | 1.36 |
| 1 | 15.6 |
| 5 | 14.6 |
| 10 | 151.7 |
| 12 | 35.9 |
| 14 | 47.2 |
| 15 | 4.3 |
| 16 | 9.8 |
| 17 | 24.5 |
| 18 | 15.1 |
| 19 | 24.3 |
| 20 | 37.7 |
| 21 | 18.3 |
| 22 | 9.3 |

### IV. Experimental conclusions:

The above data shows that the compound of the present invention has good human GHSR agonist activity.

### Test Example 2. In-vivo pharmacodynamic test of the compound of the present invention in rats

In humans and rodents, growth hormone is stimulated by growth hormone releasing hormone (GHRH) to be secreted into the circulation by the pituitary gland, which acts with the liver tissue to produce insulin-like growth factor-1 (IGF-1) and other effector factors to play a metabolic regulatory role in promoting growth and development. Therefore, a male rat model (avoiding the individual differences introduced by the menstrual period of female rats) was used to test the promotion of the compound on GH/IGF-1 signaling pathway, and to evaluate the effect of the compound of the present invention on the circulating level of growth hormone (GH).

Young male Sprague-Dawley (SD) rats (60-80 g) were purchased from Liaoning Changsheng Biotechnology Co., Ltd. (Liaoning, China). All animals were kept in a temperature-controlled room (22±2°C) with a relative humidity of 55±15% and a 12/12-h light/dark period (the light was turned off at 8: 00 pm), with 3 animals in each cage. Animals were adapted to the environment for 1 week after arrival, and allowed to free access to indicated feed and sterilized water, and the padding in the animal cage was changed twice a week. On the day before the study, the animals were fasted for 14 hrs, but allowed to free access to water.

Ibutamoren and the compound of the present invention were prepared in 10% HS15, and administered at a dosage of 4 mpk. The molar concentrations of corresponding compounds are ibuttamouren (6.4 nmol/kg), Compound 5 (5.8 nmol/kg) and Compound 14 (6.1 nmol/kg).

After basic sampling (at 0 timepoint), the compound of the present invention was orally administered to rats. Then the blood samples were collected at various time points (15 minutes, 30 minutes, and 60 minutes) after administration. Blood samples were obtained by taking blood from ophthalmic venous plexus. The blood sample was collected into an EP tube containing heparin sodium and centrifuged immediately. The plasma sample was collected and stored in a freezer at -80°C until assay. The plasma GH level in each test animal was detected by using an enzyme-linked immunosorbent assay kit from Merk-rat/Mouse Growth Hormone ELISA (USA).

### Experimental results

The effect of the compound of the present invention on GH level in blood circulation in SD rats is shown in Table 2.

**Table 2. Effect of the compound on GH concentration in Rats**

| GH concentration in rats (ng/mL) | | | |
|---|---|---|---|
| Time point (min) | **Compound No.** | | |
| | Ibutamoren | 5 | 14 |
| 0 | 12.22±9.90 | 12.23±10.44 | 9.75±11.19 |
| 15 | 54.82±28.53 | 51.12±33.98 | 70.95±40.88 |
| 30 | 20.53±9.29 | 19.07±7.17 | 21.00±12.21 |
| 60 | 11.35±4.49 | 11.76±4.14 | 14.72±5.24 |

### Experimental conclusions

When tested in the above-mentioned male rats, the GH level in the circulation of rats is significantly increased, 15 minutes after the administration of Compound 5 and Compound 14 of the present invention, and the concentration is lower than that of ibutamoren when the same effect is achieved. Compound 14 is significantly better than that of ibutamoren.

### Test Example 3. Caco-2 cell transport assay

The transport buffer in the study was 10.0 mM HEPSS in HBSS, pH 7.40±0.05. Bidirectional test of the test compound was conducted at 2.00 µM, and the final concentration of DMSO was required to be less than 1%. The cell plate was incubated for 2 hrs by standing in a CO₂ incubator at 37±1°C with 5% CO₂ and saturated humidity. All samples were mixed with acetonitrile containing an internal standard and centrifuged at 3200 xg for 10 min. The compound was tested. 100 µL supernatant was diluted with 100 µL ultrapure water, and analyzed by LC-MS/MS. The concentrations of the test compound and the control compound (Digoxin is a model verification compound, and ibutamoren is a positive control) in the initial solution, the donor solution and the receiver solution were quantified by LC-MS/MS using the peak area ratio of analyte/internal standard. After transport assay, the monolayer integrity of Caco-2 cells was determined by fluorescein exclusion assay.

**Table 3-1. Transport assay of Compound 5 of the present invention into Caco-2 cells**

| **Sample** | **Mean Pₐₚₚ (10⁻⁶ cm/s)** | | **Efflux Ratio** |
|---|---|---|---|
| | **A to B** | **B to A** | |
| Digoxin | 0.0229 | 12.0 | 523 |
| Ibutamoren | 0.0281 | 19.5 | 694 |
| Compound 5 | 0.0518 | 8.80 | 170 |

**Table 3-2. Transport assay of Compound 19 of the present invention into Caco-2 cells**

| **Sample** | **Mean Pₐₚₚ (10⁻⁶ cm/s)** | | **Efflux Ratio** |
|---|---|---|---|
| | **A to B** | **B to A** | |
| Digoxin | 0.0476 | 14.3 | 300 |
| Ibutamoren | 0.0758 | 24.1 | 318 |
| Compound 19 | 0.198 | 37.3 | 189 |

### Experimental conclusions

The above data shows that the model is successfully constructed, and the cell permeability of the compound of the present invention is significantly better than that of the reference compound ibutamoren.

### Test Example 4. Subacute toxicity test in rats

Male Sprague-Dawley (SD) rats (180-220 g) were purchased from Liaoning Changsheng Biotechnology Co., Ltd. (Liaoning, China). All animals were kept in a temperature-controlled room (22±2°C) with a relative humidity of 55±15% and a 12/12-h light/dark period (the light was turned off at 8: 00 pm), with 3 animals in each cage. Animals were adapted to the environment for 1 week after arrival, and allowed to free access to indicated feed and sterilized water, and the padding in the animal cage was changed twice a week.

The compound of the present invention was prepared in 10% HS15.

Multiple doses (30 mpk, 100 mpk and 300 mpk) of the compound of the present invention were orally administered to rats. The compound was administered once a day for consecutive 14 days. The rats were weighed twice a week. After administration, all rats were observed in a recovery period of 14 days, and weighed twice a week. Compound 5 was given by oral gavage for consecutive 14 days and then recovered for 2 weeks. There is no significant abnormality in food intake and daily behavior in each group of rats, there is no significant difference in weight change in each group of rats (FIG. 1), and there is no anatomic abnormality.

### Experimental results

The weight changes of rats in each group are shown in FIG. 1.

### Experimental conclusions

The above data shows that no obvious abnormality is observed in the subacute toxicity test of Compound 5 of the present invention in rats when the dosage was 80 times, indicating that Compound 5 is safe.

### Test Example 5. Comparison of metabolism test of Compound 5 in rats

Compound 5 was designed as a prodrug. The amount of the active ingredient ibutamoren was tested by the metabolism test in rats, so as to evaluate the advantage and disadvantage of the candidate and the positive control ibutamoren.

Experimental procedures:

6 animals were fasted for the first time for at least 12 hrs two days before administration and then uniformly given feed. Then, the animals were fasted for the second time for at least 12 hrs the day before administration, and then fed after 4 hrs. The fasting time is not exceed 20 hrs each time. The animals were allowed to free access to water during the period. Within two days before administration, the animals were adaptively trained at least once a day by touching and grabbing the animals by the same person in charge.

Before the first administration, the animals were divided into two groups according to their weight, with three animals in each group. The animals in the 1st group were given a single dose of Compound 5 as a solution by oral gavage (preparation method: medium-chain triglyceride/polyethylene glycol 1000 vitamin E succinate/ethanol/propylene glycol/water =6/2/1/1/90, 1 mg/mL). The animals in the 2nd group were given a single dose of an ibutamoren solution (water, 1 mg/mL, prepared with ibutamoren methanesulfonate) by oral gavage. The dosing volume was 3 mL/kg. The animals were weighed before administration, and the dosing volume was calculated according to the weight.

The samples were collected before administration (about -0.25 hrs) and 0.083, 0.25, 0.5, 1, 1.5, 2, 3, 5, 7 and 10 hrs after administration. The animals were anesthetized with isoflurane for a short time at each specified time point. Whole blood samples (about 0.23 mL in each group) were collected by jugular vein puncture. 50 µL of whole blood sample was quantitatively taken, added into an EP tube filled with 50 µL of pre-cooled 1mM PMSF solution in methanol, and then vortexed for 3 s. 250 uL of a precipitant containing an internal standard was immediately added, vortexed for 5 s, and centrifuged for 15 min. The supernatant was taken and analyzed by LC-MS/MS.

### Experimental results

**Table 4. Test data of drug metabolism in rats**

| **Group** | **T_{1/2} (hr)** | **Tₘₐₓ (hr)** | **Cₘₐₓ (ng/mL)** | **AUCₗₐₛₜ (hr*ng/mL)** | **AUCINF-obs (hr*ng/mL)** |
|---|---|---|---|---|---|
| **Compound 5** | 2.43±0.13 | 0.44±0.49 | 18.50±13.60 | 21.61±9.52 | 23.31±10.81 |
| Ibutamoren | 9.61±12.07 | 1.06±1.68 | 3.49±2.81 | 6.97±3.27 | 14.28±11.03 |

### Experimental conclusions

The above data show that in the drug metabolism test in rats administered by oral gavage, the Cmax and AUC of Compound 5 of the present invention are obviously superior to those of the positive control ibutamoren, and it has better drugability.

Embodiments of the present invention are described above. However, the present invention is not limited thereto. Any modifications, equivalent improvements and substitutions can be made without departing from the spirit and principle of the present invention, which are all embraced in the protection scope of the present invention.

## Claims

1. A compound of Formula I, or a racemate, a stereoisomer, a tautomer, an isotopically labeled compound, an N-oxide, a solvate, a polymorph, a metabolite, an ester, a prodrug or a pharmaceutically acceptable salt thereof: 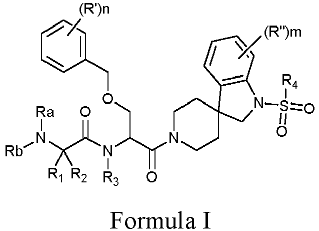 wherein
Ra is selected from H, -R, -C(=O)R or -C(=O)OR;
Rb is selected from H, -R, -C(=O)R or -C(=O)OR;
or Rb is attached to N in N-R₃ to form a ring structure 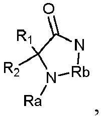 and in this case R₃ is absent, Rb is selected from 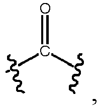
or a (C₁-C₁₂) aliphatic hydrocarbylene optionally substituted with one, two or more Rc, and Ra is as defined above;
provided that Ra and Rb are not both H;
R is selected from a (C₁-C₂₀) aliphatic hydrocarbyl group, a (C₁-C₂₀) aliphatic hydrocarbyl group optionally containing one, two or more heteroatoms, a C₃-₁₂ cycloalkyl group, a 3-12 membered heterocyclyl group, a C₆₋₁₄ aryl group or a 5-14 membered heteroaryl group, optionally substituted with one, two or more Rc;
Rc is each independently selected from halo, CN, OH, SH, =O(oxo), NH₂, -OP(=O)(OH)₂, -OP(=O)(C₁-C₁₂ alkoxy)₂, COOH, or a (C₁-C₁₂) aliphatic hydrocarbyl group, a (C₁-C₁₂) aliphatic hydrocarbyl group optionally containing one, two or more heteroatoms, a C₃₋₁₂ cycloalkyl group, a 3-12 membered heterocyclyl group, a C₆₋₁₄ aryl group or a 5-14 membered heteroaryl group, optionally substituted with one, two or more Rd;
Rd is each independently selected from halo, CN, OH, SH, =O (oxo), NH₂, -OP(=O)(OH)₂, -OP(=O)(C₁-C₁₂ alkoxy)₂, COOH, a (C₁-C₁₂) aliphatic hydrocarbyl group, and a (C₁-C₁₂) aliphatic hydrocarbyl group optionally containing one, two or more heteroatoms.
R₁, R₂, R₃, and R₄ are the same or different, and each independently selected from H, and a (C₁-C₁₂) aliphatic hydrocarbyl group optionally substituted with one, two or more Rc;
R' and R" are each independently selected from H, halo, CN, OH, SH, NH₂, - OP(=O)(OH)₂, -OP(=O)(C₁-C₁₂ alkoxy)₂, COOH, or a (C₁-C₁₂) aliphatic hydrocarbyl group and a (C₁-C₁₂) aliphatic hydrocarbyl group optionally containing one, two or more heteroatoms, optionally substituted with one, two or more Rd;
n, and m are each independently selected from 0, 1, 2, 3, 4 or 5.

2. The compound of Formula I according to claim 1, or a racemate, a stereoisomer, a tautomer, an isotopically labeled compound, an N-oxide, a solvate, a polymorph, a metabolite, an ester, a prodrug or a pharmaceutically acceptable salt thereof, wherein:
Ra is H;
Rb is selected from -R, -C(=O)R or -C(=O)OR;
R is selected from a (C₁-C₆) alkylC(=O)(C₁-C₆) alkyl- group, a HC(=O)(C₁-C₁₂) alkyl-group, a (C₁-C₁₂) alkylC(=O)OCH(C₁-C₁₂ alkyl)- group, (C₁-C₁₂) alkyl-, a 5-14 membered heteroaryl-(C₁-C₁₂) alkyl- group, and a 5-14 membered heteroaryl-(C₁-C₁₂) alkyl- group, optionally substituted with one, two or more NH₂;
or R is selected from a (C₁-C₁₂) alkyl group, a (C₁-C₁₂) alkoxy group, and a (C₂-C₂₀) alkenyl group;
or R is selected from a (C₁-C₁₂) alkyl group and a (C₁-C₁₂) alkoxy group optionally substituted with one, two, or more -OP(O)(OH)₂;
Or R is selected from a (C₁-C₁₂) alkyl group optionally substituted with one, two, or more -OP(O)(C₁-C₁₂ alkoxy)₂;
or R is selected from 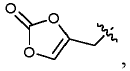 a (C₁-C₆) alkylC(=O)(C₂-C₆) alkenyl- group, and a 5-14 membered heteroaryl group, optionally substituted with one, two, or more (C₁-C₆) alkyl;
or R is selected from a (C₁-C₁₂) alkylC(=O)OCH(C₁-C₁₂ alkyl)- group, a C₃₋₁₂ cycloalkylC(=O)OCH(C₁-C₁₂ alkyl)- group, a (C₁-C₁₂) alkylC(=O)OCH₂- group, and a C₃₋₁₂ cycloalkylC(=O)OCH(C₁-C₁₂ alkyl)- group.

3. The compound of Formula I according to claim 1 or 2, or a racemate, a stereoisomer, a tautomer, an isotopically labeled compound, an N-oxide, a solvate, a polymorph, a metabolite, an ester, a prodrug or a pharmaceutically acceptable salt thereof, wherein:
the (C₁-C₂₀) aliphatic hydrocarbyl group is selected from a (C₁-C₂₀) alkyl group, a (C₂-C₂₀) alkenyl group, and a (C₂-C₂₀)alkynyl group; and preferably, the "(C₁-C₂₀) aliphatic hydrocarbyl group" is selected from a (C₁-C₁₂) alkyl group, a (C₂-C₁₂) alkenyl group, and a (C₂-C₁₂)alkynyl group; and the (C₁-C₁₂) aliphatic hydrocarbyl group is selected from a (C₁-C₆) alkyl group, a (C₂-C₆) alkenyl group, and a (C₂-C₆)alkynyl group; and
preferably, Rb is attached to N in N-R₃ to form a ring structure 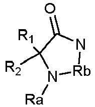 that is further preferably: 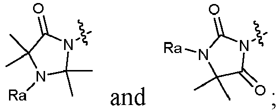 and in this case R₃ is absent, and R₁, R₂ and Ra are as defined in claim 1 or 2.

4. The compound of Formula I according to any one of claims 1 to 3, or a racemate, a stereoisomer, a tautomer, an isotopically labeled compound, an N-oxide, a solvate, a polymorph, a metabolite, an ester, a prodrug or a pharmaceutically acceptable salt thereof, wherein:
R is selected from methyl, ethyl, n-propyl, iso-propyl, n-butyl, iso-butyl, t-butyl, n-pentyl, iso-pentyl, neo-pentyl, n-hexyl, ethenyl, 1-propenyl, 2-propenyl, 1-methylethenyl, 1-butenyl, 1-ethylethenyl, 1-methyl-2-propenyl, 2-butenyl, 3-butenyl, 2-methyl-1-propenyl, 2-methyl-2-propenyl, 1-pentenyl, 1-hexenyl, ethynyl, 1-propynyl, 2-propynyl, 1-butynyl, 1-methyl-2-propynyl, 3-butynyl, 1-pentynyl, 1-hexynyl, cyclopropyl, cyclobutyl, cyclopentyl, cyclohexyl, NH₂CH₂-, 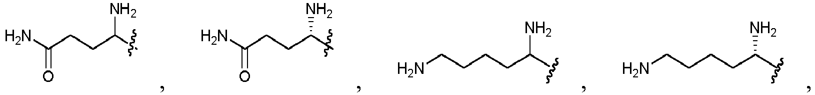 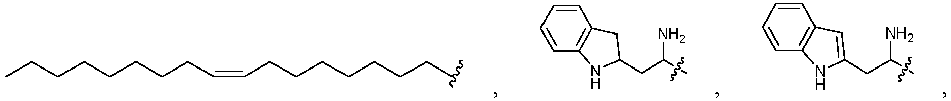 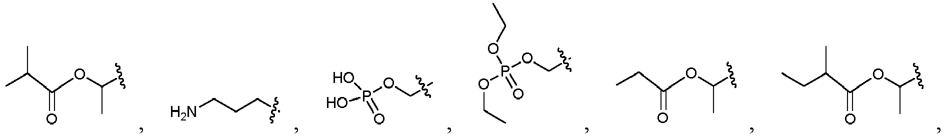 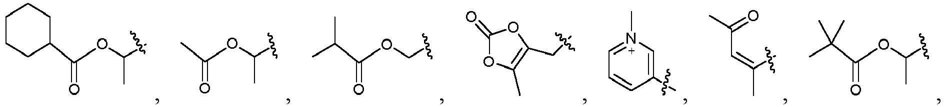 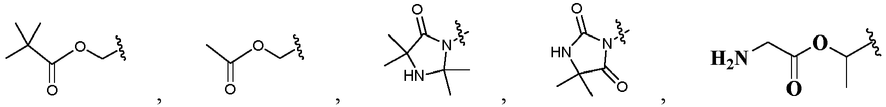 or 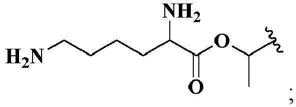 and wherein R is optionally further substituted with one, two or more Rc.

5. The compound of Formula I according to any one of claims 1 to 4, or a racemate, a stereoisomer, a tautomer, an isotopically labeled compound, an N-oxide, a solvate, a polymorph, a metabolite, an ester, a prodrug or a pharmaceutically acceptable salt thereof, wherein the compound of Formula I is further preferably a compound of Formula II: 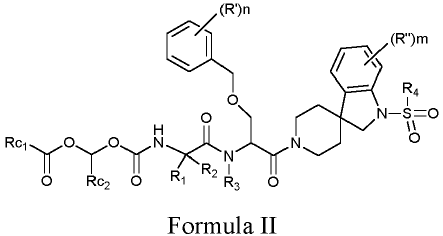
wherein Rc₁ and Rc₂ are the same or different, and each independently selected from H or Rc; and
Rc, R', R", R₁, R₂, R₃, R₄, n, and m are as defined in claim 1.

6. The compound of Formula I according to any one of claims 1 to 5, or a racemate, a stereoisomer, a tautomer, an isotopically labeled compound, an N-oxide, a solvate, a polymorph, a metabolite, an ester, a prodrug or a pharmaceutically acceptable salt thereof, wherein the compound of Formula I is further preferably a compound of Formula III: 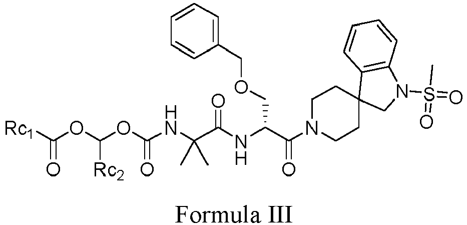 wherein Rc₁ and Rc₂ are the same or different, and each independently selected from H or Rc; and Rc is as defined in claim 1.

7. The compound of Formula I according to any one of claims 1 to 6, or a racemate, a stereoisomer, a tautomer, an isotopically labeled compound, an N-oxide, a solvate, a polymorph, a metabolite, an ester, a prodrug or a pharmaceutically acceptable salt thereof, wherein the compound of Formula I is further preferably a compound of Formula IIIA or Formula IIIB: 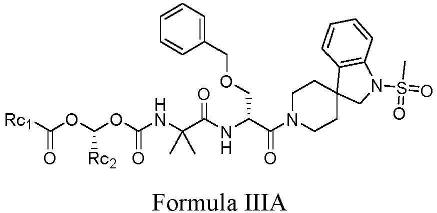 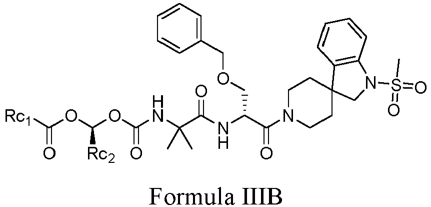 wherein Rc₁ and Rc₂ are the same or different, and each independently selected from Rc; and Rc is as defined in claim 1.

8. The compound of Formula I according to any one of claims 5 to 7, or a racemate, a stereoisomer, a tautomer, an isotopically labeled compound, an N-oxide, a solvate, a polymorph, a metabolite, an ester, a prodrug or a pharmaceutically acceptable salt thereof, wherein
Rc₁ is selected from a C₁-C₁₂ alkyl group, a C₃-C₈ cycloalkyl group, and a C₁-C₁₂ alkyl group optionally substituted with one, two, or more NH₂;
preferably Rc₁ is selected from methyl, ethyl, iso-propyl, n-propyl, n-butyl, iso-butyl, t-butyl, aminomethyl, 1,5-diaminon-pentyl, cyclopropyl, cyclobutyl, cyclopentyl or cyclohexyl;
Rc₂ is selected from H or a C₁-C₁₂ alkyl group; and
preferably Rc₂ is selected from H or methyl.

9. The compound of Formula I according to any one of claims 1 to 8, or a racemate, a stereoisomer, a tautomer, an isotopically labeled compound, an N-oxide, a solvate, a polymorph, a metabolite, an ester, a prodrug or a pharmaceutically acceptable salt thereof, having a structure shown below: 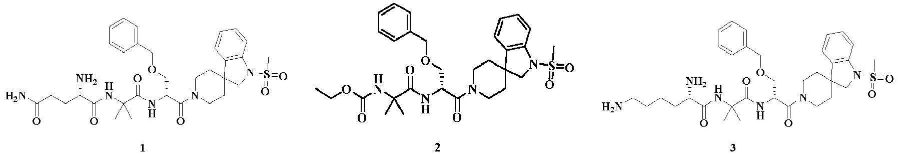 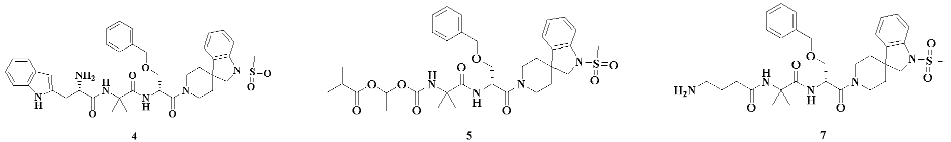 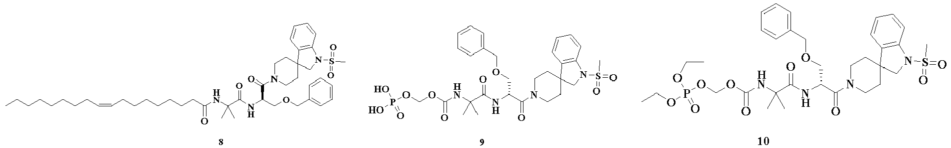 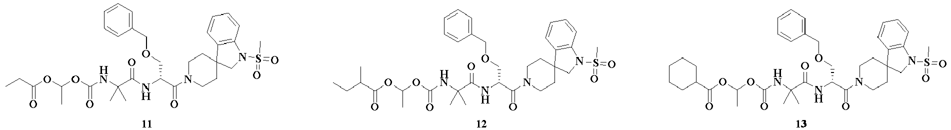 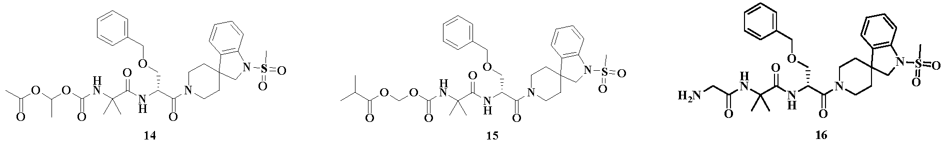 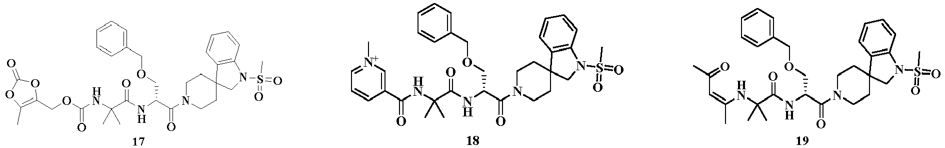 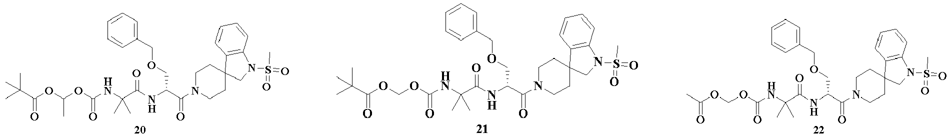 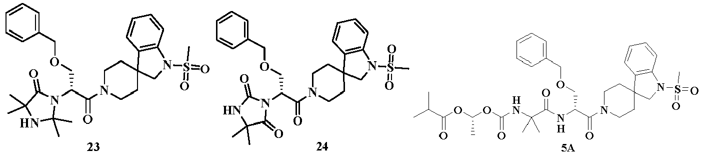 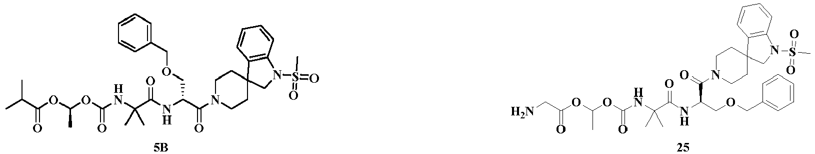 and

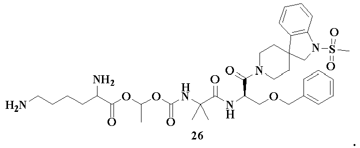

10. A method for preparing the compound of Formula I according to any one of claims 1 to 9, or a racemate, a stereoisomer, a tautomer, an isotopically labeled compound, an N-oxide, a solvate, a polymorph, a metabolite, an ester, a prodrug or a pharmaceutically acceptable salt thereof, the method comprising the following steps:
reacting ibutamoren or a derivative thereof with a raw material capable of introducing the modifying group Ra and/or Rb in a suitable reagent under suitable conditions, to obtain an amino modified product; and optionally adding a protecting group and removing a protecting group under suitable conditions;
or the method comprising the following steps: reacting ibutamoren or a derivative thereof in the presence of potassium dihydrogen phosphate and acetone under suitable conditions;
or the method comprising the following steps: reacting ibutamoren or a derivative thereof in the presence of a chloroformate (or a substituted thiocarbonate) and a base under suitable conditions, where the base is selected from triethylamine, and the solvent used in the reaction is dichloromethane;
or the method comprising the following steps: undergoing a condensation reaction with an amino acid to form an amide in the presence of a condensing agent and a base, where the condensing agent is selected from 2-(7-azabenzotriazole)-N,N,N',N'-tetramethyluronium hexafluorophosphate and dicyclohexylcarbodiimide, and the base is selected from triethylamine.

11. A pharmaceutical composition, comprising the compound of Formula I according to any one of claims 1 to 9, or a racemate, a stereoisomer, a tautomer, an isotopically labeled compound, an N-oxide, a solvate, a polymorph, a metabolite, an ester, a prodrug or a pharmaceutically acceptable salt thereof.

12. Use of the compound of Formula I according to any one of claims 1 to 9, or a racemate, a stereoisomer, a tautomer, an isotopically labeled compound, an N-oxide, a solvate, a polymorph, a metabolite, an ester, a prodrug or a pharmaceutically acceptable salt thereof, or the pharmaceutical composition according to claim 11 in the preparation of GHSR agonists or in the preparation of drugs for diagnosing, preventing and/or treating growth hormone dependent diseases, wherein preferably, the diseases or disorders are associated with growth hormone deficiency or growth hormone dependence; and preferably, the compound promotes the plasma level of growth hormone in human and animals after administration.
